# EUROPEAN PATENT APPLICATION

(11) **EP 1 048 659 A1**
(43) Date of publication of application: **02.11.2000**
(21) Application number: 98953024.1
(22) Date of filing: 12.11.1998
(51) Int. Cl.: C07D 277/34, C07D 417/10, A61K 31/425, A61K 31/55

(54) **RETINOID RECEPTOR AGONISTS**

(30) Priority: 12.11.1997 JP 31083597
(71) Applicant: Institute of Medicinal Molecular Design, Inc., Tokyo 113-0033 (JP)
(72) Inventor: KAGECHIKA, Hiroyuki, Tokyo 161-0062 (JP); HASHIMOTO, Yuichi, Tokyo 161-0032 (JP); ITAI, Akiko, Tokyo 113-0033 (JP)
(74) Representative: Sternagel, Fleischer, Godemeyer & Partner Patentanwälte
(86) International application number: JP9805091
(87) International publication number: WO9924415

(57) **Abstract**

The invention provides a substance acting on a retinoid receptor which has a retinoid-like activity or an action of controlling retinoid activities, for example, enhancement or suppression.

Compounds represented by the following general formula (I) wherein R¹, R², R³, R⁴, and R⁵ independently represent hydrogen atom or a lower alkyl group, or two adjacent groups selected therefrom may combine together with carbon atoms of the phenyl ring to which they bind to form a 5- or 6-membered ring which may optionally be substituted with one or more alkyl groups; and X represents a group represented by -C(R⁶)=CH-, -CH=C(R⁷)-, -N(R⁸)-CO-, -CO-N(R⁹)-, -C(=CHR¹⁰), -CO-, or -NR¹¹-wherein R⁶, R⁷, R⁸, R⁹, R¹⁰, and R¹¹ independently represent hydrogen atom or a lower alkyl group.

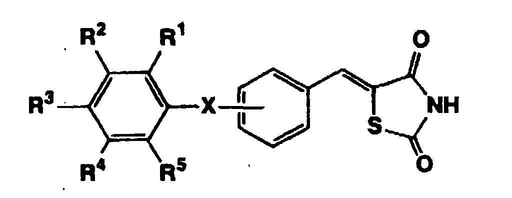

## Description

### Technical Field

The present invention relates to substances acting on a retinoid receptor that have physiological activities similar to those of retinoids such as retinoic acid or controlling activities on retinoid actions, and medicaments comprising said compound as an active ingredient.

### Background Art

Retinoic acid (vitamin A acid), an active metabolite of vitamin A, has extremely important physiological functions, e.g., inducing differentiation of immature cells under development processes toward mature cells having specific functions, enhancement of cell proliferation, and life support action. It has been revealed that various vitamin A derivatives synthesized so far also have similar physiological functions, for example, the benzoic acid derivatives disclosed in Japanese Patent Unexamined Publication (KOKAI) Nos. (Sho)61-22047/1986 and (Sho)61-76440/1986, and the compounds described in Journal of Medicinal Chemistry, 1988, Vol. 31, No. 11, p.2182. "Retinoids" is a general term for retinoic acid and the aforementioned compounds having retinoic acid-like biological activities.

For example, it was proved that all trans retinoic acid binds as a ligand to the retinoic acid receptor (RAR) present in cellular nucleus, which belongs to the intranuclear receptor super family (Evans, R.M., Science, 240, p.889, 1988), and regulates proliferation and differentiation of animal cells or cellular mortalities (Petkovich, M., et al., Nature. 330, pp.444-450, 1987). It has also been suggested that the aforementioned compounds having the retinoic acid-like biological activities, e.g., 4-[(5,6,7,8-tetrahydro-5,5,8,8-tetramethyl-2-naphthalenyl)carbamoyl]benzoic acid: Am80, also bind to RAR in similar manners to retinoic acid to exhibit their physiological actions (see, Hashimoto, Y., Cell Struct. Funct., 16, pp.113-123, 1991; Hashimota, Y., et al., Biochem. Biophys. Res. Commun., 166, pp.1300-1307, 1990).

Clinically, these compounds were found as useful for therapeutic and preventive treatments of vitamin A deficiency disease, hyperkeratosis of epithelial tissue, rheumatism, delayed allergy, bone diseases, leukemia and certain types of cancer. However, because of variety of biological activities of these retinoids, they are not fully satisfactory medicaments from a viewpoint of side effect. Therefore, it has been desired to create retinoids having characteristic activities and molecules controlling their activities.

As agents for controlling the activities of retinoids, there have been known benzodiazepine derivatives such as 4-[5H-2,3-(2,5-dimethyl-2,5-hexano)-5-methyldibenzo[b,e][1,4]diazepin-11-yl]benzoic acid and 4-[1,3-dihydro-7,8-(2,5-dimethyl-2,5-hexano)-2-oxo-2H-1,4-benzodiazepin-5-yl]benzoic acid (PCT/JP96/2709, International Publication WO97/11061). Although these compounds, per se, have no retinoid action or their retinoid actions are negligible, they have an action of remarkably enhancing retinoids such as retinoic acid. Therefore, they have been suggested to be useful for therapeutic and preventive treatments of vitamin A deficiency disease, hyperkeratosis of epithelial tissue, rheumatism, delayed allergy, bone diseases, leukemia, and certain types of cancer.

As for expression of physiological activities of retinoic acid, existence of retinoid X receptor (RXR, of which ligand is 9-cis-retinoic acid) has been verified. It has been revealed that the retinoid X receptor forms a dimer with the retinoic acid receptor (RAR) to induce or suppress gene transcriptions, thereby controls the expression of the physiological activities of retinoic acid (Mangelsdorf, D.J. et al., Nature, 345, pp.224-229). It has also been revealed that the retinoid X receptor (RXR) hinds to the intranuclear receptor of active vitamin D₃, PPAR whose involvement in lipid metabolism is suggested, and other receptors as well as the retinoic acid receptor (RAR), thereby controls expression of actions of physiologically active substances binding to these receptors. e.g. vitamin D₃, thyroxine and the like (Mangelsdorf, D.J. et al., The Retinoids, 2nd Ed., Ravan Press, pp.319-350, 1994).

As agents for controlling retinoid actions, compounds are also known to exist which have antagonistic action on retinoids and attenuate typical actions of the above retinoids (Eyrolles, L., et al., Journal of Medicinal Chemistry, 37(10), pp.1508-1517, 1994). This reference discloses that some compounds such as 4-(5H-7,8,9,10-tetrahydro-5,7,7,10,10-pentamethylbenzo[e]naphtho[2,3-b][1,4]diazepin-13-yl)benzoic acid act as antagonists of retinoids. Moreover, certain compounds including 4-(13H-10,11,12,13-tetrahydro-10,10,13,13,15-pentamethylnaphtho[2,3-b][1,2-e][1,4]diazepin-7-yl)benzoic acid have been found as a retinoid antagonist by the inventor of the present invention (specification of Japanese Patent Application No. (Hei)7-255912/1995).

It has been considered that the carboxyl groups of the retinoids such as retinoic acid and Am80, or the carboxyl groups of the aforementioned retinoid activity-enhancing compounds and the retinoid antagonists are essential functional groups for each desired biological activity. For example, it has been known that the desired biological activities are lost by the substitution of carboxyl group with a functional group such as sulfonamide and tetrazole. Some compounds having a thiazolidine skeleton such as ciglitazone and troglitazone have been suggested to act on the γ-subtype of PPAR (peroxisome proliferator-activated receptor) belonging to the intranuclear receptor super family. However, it has not been known that compounds obtained by replacing the carboxyl groups of the aforementioned physiologically active compounds with a thiazolidine ring interact with retinoid receptors to exhibit physiological activities.

As thiazolidinedione derivatives, N-benzyl-type 2,4-thiazolidinedione derivatives having hypoglycemic action have been known (Japanese Patent Unexamined Publication (KOKAI) No. (Hei)9-48771/1997; and Abstract of the 17th Medicinal Chemistry Symposium and the 6th Annual Meeting of Medicinal Chemistry Section, pp.114-115, 1-P-30, October 27, 1997, published by the Pharmaceutical Society of Japan). However, the references neither suggest nor teach that these thiazolidinedione derivatives have retinoid-like actions, or they act as an agent for controlling retinoid activities.

### Disclosure of the Invention

An object of present invention is to provide substances acting on the retinoid receptor which have a retinoid-like activity or an action of controlling the retinoid activities (for example, enhancing or suppressing activities on retinoid). Another object of the present invention is to provide medicaments which comprise said compound as an active ingredient.

The inventor of the present invention conducted various studies to achieve the foregoing objects, and as a result, they found that thiazolidine compounds represented by the following general formulas had retinoic acid-like biological activities, or an action for enhancing or suppressing the activities of retinoids. The present invention was achieved on the basis of these findings.

The present invention thus provides compounds represented by the following general formula (I): 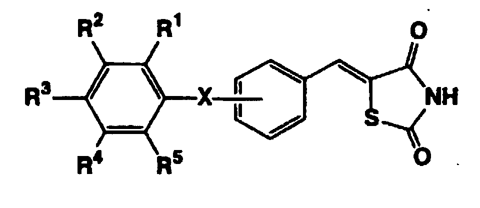 wherein R¹, R², R³, R⁴, and R⁵ independently represent hydrogen atom or a lower alkyl group, or two adjacent groups selected therefrom may combine together with carbon atoms of the phenyl ring to which they bind to form a 5- or 6-membered ring which may optionally be substituted with one or more alkyl groups; and X represents a group represented by -C(R⁶)=CH-, CH=C(R⁷)-, -N(R⁸)-CO-, -CO-N(R⁹)-, -C(=CHR¹⁰), -CO-, or -NR¹¹- wherein R⁶, R⁷, R⁸, R⁹, R¹⁰, and R¹¹ independently represent hydrogen atom or a lower alkyl group, and compounds represented by the following general formula (II): 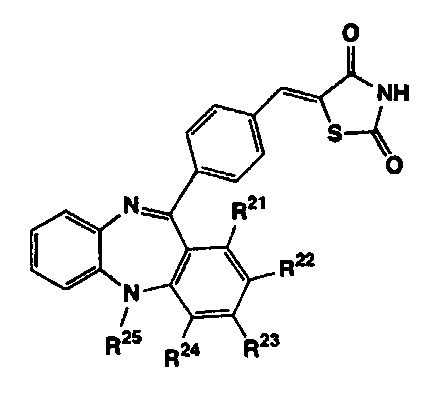 wherein R²¹, R²², R²³, and R²⁴ independently represent hydrogen atom or a lower alkyl group, or two adjacent groups selected therefrom may combine together with carbon atoms of the phenyl ring to which they bind to form a 5- or 6-membered ring which may optionally be substituted with one or more alkyl groups; and R²⁵ represents hydrogen atom or a lower alkyl group, or salts thereof.

According to another aspect of the present invention, there are provided medicaments comprising the compounds represented by aforementioned general formulas or physiologically acceptable salts thereof, or hydrates thereof or solvates thereof. These medicaments are useful as agents having retinoid-like actions or agents for controlling retinoid activities (preferably agents for enhancing retinoid activities or those for suppressing retinoid activities).

According to further aspects of the present invention, there are provided use of the aforementioned substances for the manufacture of the aforementioned medicaments; and methods for therapeutic and/or preventive treatments of a disease in which a receptor belonging to the intranuclear receptor super family (Evans, R.M., Science, 240, p.889, 1988), preferably a retinoid receptor (RAR and/or RXR) is involved, which comprises the step of administering an effective amount of the aforementioned substances to a mammal including a human.

### Most Preferred Embodiments to Carry out the Invention

In the aforementioned general formula (I), R¹, R², R³, R⁴, and R⁵ independently represent hydrogen atom or a lower alkyl group. As the lower alkyl group, a linear or branched alkyl group having about 1 to 6 carbon atoms, preferably 1 to 4 carbon atoms can be used. For example, methyl group, ethyl group, n-propyl group, isopropyl group, n-butyl group, sec-butyl group, tert-butyl group and the like may be used.

Two adjacent groups selected from the group consisting of R¹, R², R³, R⁴, and R⁵ may combine together with carbon atoms of the phenyl ring to which they bind to form one or two, preferably one 5- or 6-membered ring which may optionally be substituted with one or more alkyl groups. As the alkyl group that may be present on the ring(s), a linear or branched alkyl group having about 1 to 6 carbon atoms, preferably 1 to 4 carbon atoms may be used. For example, methyl group, ethyl group and the like may be used, and preferably 2 to 4 methyl groups, more preferably four methyl groups, may be present on the ring(s). For example, R² and R³ together with the phenyl ring to which R² and R³ bind may preferably form 5,6,7,8-tetrahydronaphthalene ring, 5,5,8,8-tetramethyl-5,6,7,8-tetrahydronaphthalene ring or the like.

X represents any one of groups represented by -C(R⁶)=CH-, -CH=C(R⁷)-, -N(R⁸)-CO-, -CO-N(R⁹)-, -C(=CHR¹⁰), -CO-, or -NR¹¹-. In these groups, R⁶, R⁷, R⁸, R⁹, R¹⁰, and R¹¹ independently represent hydrogen atom or a lower alkyl group. As the lower alkyl group, for example, a linear or branched alkyl group having 1 to 4 carbon atoms may be used. More specifically, methyl group, ethyl group and the like are preferably used. The substituting position of X on the phenyl group of the benzylidenethiazolidinedione moiety is not particularly limited. Para-substitution or meta-substitution may be preferred.

In the aforementioned general formula (II), R²¹, R²², R²³ and R²⁴ independently represent hydrogen atom or a lower alkyl group. As the lower alkyl group, a linear or branched alkyl group having about 1 to 6 carbon atoms, preferably 1 to 4 carbon atoms may be used. For example, methyl group, ethyl group, n-propyl group, isopropyl group, n-butyl group, sec-butyl group, tert-butyl group and the like may be used. R²⁵ represents hydrogen atom or a lower alkyl group. As the lower alkyl group, for example, a linear or branched alkyl group having 1 to 4 carbon atoms may be used. More specifically, methyl group, ethyl group and the like may preferably be used.

Two adjacent groups selected from the group consisting of R²¹, R²², R²³, and R²⁴ may combine together with carbon atoms of the phenyl ring to which they bind to form one or two, preferably one 5- or 6-membered ring which may optionally be substituted with one or more alkyl groups. As the alkyl group that may be present on the ring(s), a linear or branched alkyl group having about 1 to 6 carbon atoms, preferably 1 to 4 carbon atoms, may be used. For example, methyl group, ethyl group and the like may be used, and preferably 2 to 4 methyl groups, more preferably four methyl groups, may be present on the ring(s). For example, R²² and R²³ together with the phenyl ring to which R²² and R²³ bind may preferably form 5,6,7,8-tetrahydronaphthalene ring or 5,5,8,8-tetramethyl-5,6,7,8-tetrahydronaphthalene ring.

The aforementioned compounds may form base addition salts. For example, they may exist as metal salts such as sodium salts, potassium salts, magnesium salts and calcium salts, ammonium salts, organic amine salts such as triethylamine salts and ethanolamine salts and the like. Among those salts, physiologically acceptable salts can be used as the active ingredients of the medicaments of the present invention. The aforementioned compounds may have one or two asymmetric carbon atoms depending on the types of substituents. Any optical isomers based on the asymmetric carton atom(s), any mixtures of optical isomers, racemates, diastereomers based on two or more asymmetric carbon atoms, any mixtures of the diastereomers and the like fall within the scope of the present invention. Furthermore, geometrical isomers based on cis- or trans-configuration of one or more double bonds and any mixtures of the geometrical isomers, and any hydrates and solvates of the compounds in free form or the compounds in the forms of salts also fall within the scope of the present invention.

Among the compounds of the present invention, preferred compounds include those listed below. However, the compounds of the present invention or compounds that can be utilized as the active ingredients of the medicaments of the present invention are not limited to the following compounds. In the following description, "para" and "meta" indicate that the substituting position of X on the phenyl group of the benzylidenethiazolidinedione moiety is para-position or meta-position, respectively, and Me represents methyl group.

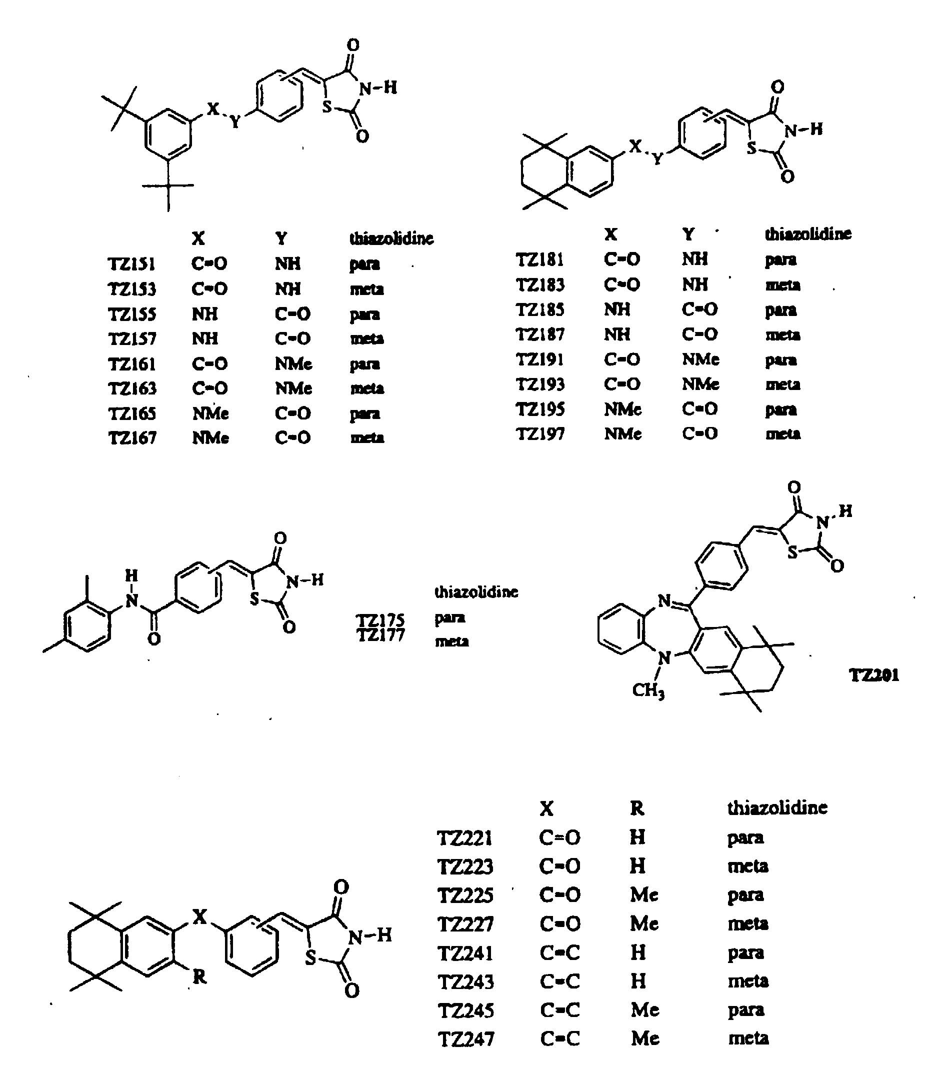

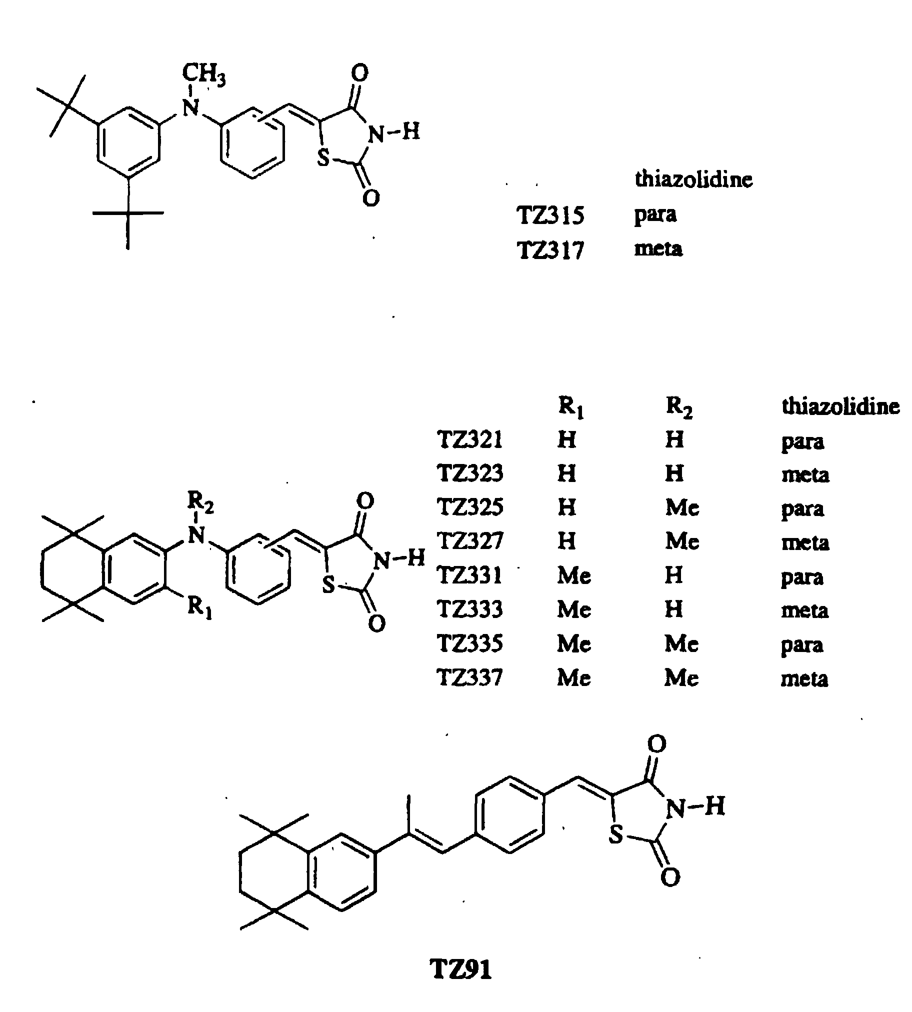

As for the preparations of the compounds of the aforementioned formulas (I) and (II), synthetic examples of the aforementioned typical compounds are specifically detailed in the examples given in the specification. Therefore, those skilled in the art will be able to readily prepare any compounds falling within the compounds of the present invention represented by the aforementioned formulas (I) and (II) by referring to those examples, or if necessary, appropriately modifying or altering the disclosed methods.

The compounds of the aforementioned formulas (I) and (II) can interact with a retinoid receptor (the term "retinoid receptor" used in the specification encompasses the retinoic acid receptors RAR and RXR, and the term means one or more of receptors with which a retinoid such as retinoic acid can interact), and they, per se, exhibit a retinoid-like physiological activities as an agonist, or have an action for enhancing or suppressing the physiological activities of retinoids.

Therefore, the medicaments comprising the aforementioned compound as an active ingredient are useful as agents having retinoid-like activities or agents for controlling retinoid activities. Which of the actions the compound of the aforementioned formula (I) or (II) possesses can be easily determined by a method described in detail in the examples of the specification. An evaluation for compounds enhancing retinoid activities is described in International Publication WO97/11061 (PCT/JP96/2709), and an evaluation for compounds suppressing retinoid activities is described in Eyrolles, L., et al., Journal of Medicinal Chemistry, 37 (10), pp.1508-1517, 1994, and the specification of Japanese Patent Application No. (Hei)7-255912/1995.

Among the aforementioned compounds, those exhibiting retinoid-like activities have, for example, cell differentiation activity, cell proliferation enhancing activity, life supporting activity and the like, and they can be used as active ingredients of medicaments for preventive or therapeutic treatments of vitamin A deficiency disease, hyperkeratosis of epithelial tissue, psoriasis, allergic diseases, immunological diseases such as rheumatism, bone diseases, leukemia, or cancers.

Among the aforementioned compounds, those enhancing retinoid activities, per se, have substantially no retinoid-like activity, or they have slight or moderate retinoid-like activities. However, where those compounds are allowed to coexist with a retinoid such as retinoic acid, the physiological activities of the retinoid (typical examples include cell differentiation activity, cell proliferation enhancing activity, life supporting activity and the like) are remarkably enhanced.

Although it is not intended to be hound by any specific theory, where the compound enhancing retinoid activities, per se, exhibit retinoid activities, synergistic actions are achieved. Therefore, where retinoids such as retinoic acid or the aforementioned compounds having retinoic acid-like biological activities (for example, 4-[(5,6,7,8-tetrahydro-5,5,8,8-tetramethyl-2-naphthalenyl)carbamoyl]benzoic acid: Am80) are administered as medicaments for the preventive or therapeutic treatments of vitamin A deficiency disease, hyperkeratosis of epithelial tissue, psoriasis, allergic diseases, immunological diseases such as rheumatism, bone diseases, leukemia, or cancers, the compounds enhancing retinoid activities can be used as agents that enhance the activities of the retinoids.

Even when retinoids are not administered for the preventive and therapeutic treatments of the aforementioned diseases, the compounds enhancing retinoid activities can increase the activities of retinoic acid that inherently exists in living bodies, and thus the compounds may be administered as medicaments for the purpose of the preventive and therapeutic treatments of the aforementioned diseases. Furthermore, the aforementioned compounds may be used, in addition to the enhancement of the activities of retinoids, to enhance activities of physiologically active substances such as, for example, steroid compounds, vitamin D compounds including vitamin D₃, thyroxine and the like which bind to receptors belonging to the intranuclear receptor super family present in cellular nucleus to exhibit their physiological activities (Evans, R.M., Science, 240, p.889, 1988). They are useful as medicaments for preventive or therapeutic treatments of, for example, diabetes, arteriosclerosis, hyperlipidemia, hypercholesterolemia, bone diseases, rheumatism, immunological diseases and the like.

As the intranuclear receptors, for example, the intranuclear receptor for active vitamin D₃, the PPAR involved in lipid metabolism, the thyroxine receptor, the COUP and the like have been known (as for these receptors, see, Mangelsdorf, D.J. et al., The Retinoids, 2nd Ed., Ravan Press, pp.319-350, 1994). It has been revealed that these receptors bind to the retinoid X receptor (RXR) to have the aforementioned physiologically active substances exhibit their activities.

Among the aforementioned compounds, those suppressing retinoid activities have an action of markedly suppressing the physiological activities of retinoids (cell differentiation activity, cell proliferation enhancing activity, life supporting activity and the like). Although it is not intended to be bound by any specific theory, it is believed that compounds having such an action bind to retinoid X receptor (RXR), which forms a dimer with the retinoic acid receptor (RAR), thereby control the expression of the physiological activity of retinoids such as retinoic acid. These compounds are useful for preventive and/or therapeutic treatments of endogenous hypervitaminosis of vitamin A caused by excessive vitamin A in vitro, or exogenous hypervitaminosis of vitamin A caused by retinoic acid or a compound having retinoic acid-like biological activities (e.g., 4-[(5,6,7,8-tetrahydro-5,5,8,8-tetramethyl-2-naphthalenyl)carbamoyl]benzoic acid: Am80 or the like) which is administered for therapeutic or preventive treatment of vitamin A deficiency disease, hyperkeratosis of epithelial tissue, psoriasis, allergic diseases, immunological diseases such as rheumatism, bone diseases, leukemia, or cancers.

Cancers such as leukemia can be treated by administering the compounds suppressing retinoid activities, per se, alone or in combination with other retinoid or an antitumor agent. The aforementioned compounds can suppress activities of substances that bind to a receptor belonging to the intranuclear receptor super family present in the nucleus of cells (Evans, R.M., Science, 240, p.889, 1988) to express physiological activities, for example, steroid compounds, vitamin D compounds such as vitamin D₃, thyroxine and orphan receptors whose ligands are unknown. Accordingly, the aforementioned compounds can also be used for controlling the expression of the physiological activities of these substances. The compounds suppressing retinoid activities which bind to the retinoid X receptor (RXR) can be thus used for, for example, preventive and/or therapeutic treatments of diseases with abnormalities of biological activities in which one or more of receptors belonging to the intranuclear receptor super family involve.

The medicament of the present invention comprises, as an active ingredient, at least one substance selected from the group consisting of the compounds represented by the, aforementioned formula (I) and salts thereof, and hydrates thereof and solvates thereof, and substances selected from the group consisting of the compounds represented by the aforementioned formula (II) and salts thereof, and hydrates thereof and solvates thereof. As the medicament of the present invention, the aforementioned substance, per se, may be administered. However, a pharmaceutical composition for oral administration or parenteral administration may preferably be administered which can be prepared by a method well known to those skilled in the art. Examples of the pharmaceutical compositions suitable For oral administrations include, for example, tablets, capsules, powders, subtilized granules, granules, liquids, syrups and the like. Examples of the pharmaceutical compositions suitable for parenteral administrations include, for example, injections, suppositories, inhalants, eye drops, nasal drops, ointments, creams, patches and the like.

The aforementioned pharmaceutical compositions may be prepared by the addition of pharmacologically and pharmaceutically acceptable additives. Examples of pharmacologically and pharmaceutically acceptable additives include, (or example, excipients, disintegrators and disintegrating aids, binders, lubricants, coating agents, colorants, diluents, base materials, dissolving agents and dissolving aids, isotonic agents, pH modifiers, stabilizers, propellants, adhesives and the like.

The doses of the medicaments of the present invention are not particularly limited, and they can suitably be selected depending on nature of the action, strength of the action and the like, and also can suitably be increased or decreased depending on various factors to be generally considered, for example, the body weight or age of a patient, the kind or symptom of a disease, an administration route and the like. In general, as for the medicaments containing compounds having retinoid-like activities as active ingredients, doses may be appropriately chosen so as to be similar to the doses of retinoic acid used as a medicament, or by making reference to the doses. For example, for oral administrations, they may be used in a dose of from 0.01 to 1,000 mg per day for an adult. Also as for the medicaments comprising the compounds enhancing retinoid activities or suppressing retinoid activities, doses can be similarly chosen. For example, for oral administrations, the medicament may be used in a dose of from 0.01 to 1,000 mg per day for an adult.

### Examples

The present invention will be more specifically explained with reference to the following examples. However, the scope of the present invention is not limited to these examples. The compound numbers in the examples correspond to those of the compounds described above as preferred examples and those in the synthetic schemes shown below.

### Example 1: Synthesis of TZ91

4-[2-(5,6,7,8-Tetramethyl-5,5,8,8-tetrahydro-2-naphthyl)propenyl]benzaldehyde (24 mg, 0.072 mmol), 2,4-thiazolidinedione (10 mg, 0.085 mmol) and piperidine (5 mg, 0.058 mmol) were dissolved in ethanol (2.5 ml) and the solution was refluxed overnight. The reaction mixture was poured into 1 N hydrochloric acid and extracted with ethyl acetate. The organic layer was washed with water and dehydrated over Na₂SO₄. The solvent was evaporated, and the residue was recrystallized from methanol to obtain TZ91 (quantitative).
TZ91: Yellow needles (methanol); mp 227-229°C; ¹H-NMR (400 MHz, CDCl₃): 8.24 (br s, 1H), 7.87 (s, 1H), 7.51 (d, 2H, J=8.8Hz), 7.48 (d, 2H, J=8.8Hz), 7.45 (d, 1H, J=1.5Hz), 7.33 (d, 1H, J=8.4Hz), 7.30 (dd, 1H, J=8.4, 1.8Hz), 6.78 (br s, 1H), 2.32 (d, 3H, and J= 1.5Hz), 1.71 (s, 4H), 1.34 (s, 6H), 1.31 (s, 6H); Anal. Calcd. for C₂₇H₂₉NO₂S, C: 75.15%, H: 6.77%, N, 3.25%; Found, C: 75.08%, H: 6.97%, N, 3.11%.

### Example 2: Synthesis of TZ151

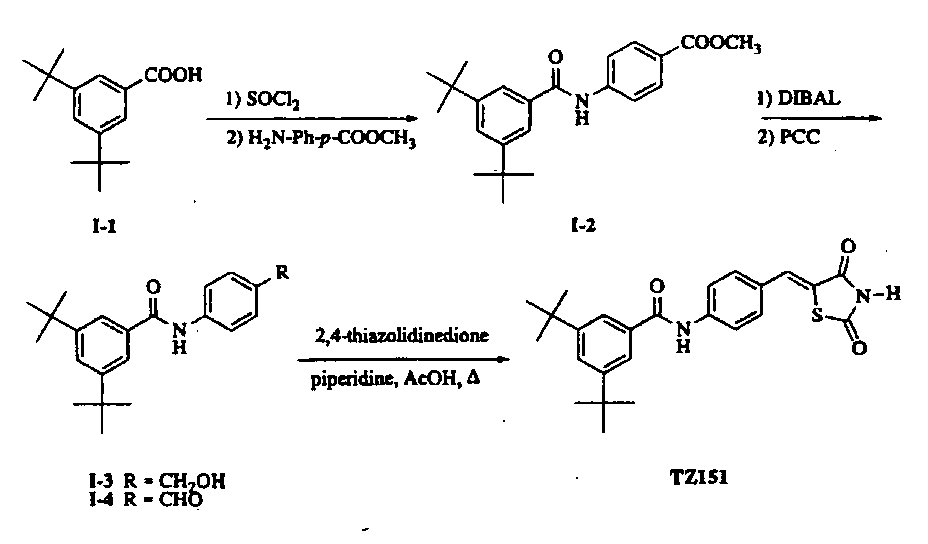

3,5-Di-tert-butylbenzoic acid (I-1) (1.00 g, 4.27 mmol) was suspended in thionyl chloride (2.50 g, 21.0 mmol) and anhydrous benzene (12 ml) and the mixture was refluxed for 14 hours. Thionyl chloride was evaporated, and the residue was added with methyl p-aminobenzoate (645 mg, 4.27 mmol), and then suspended in anhydrous benzene (30 ml) and anhydrous pyridine (1 ml). The mixture was stirred at room temperature for 1.5 hours. The reaction mixture was added with ice water and 2 N hydrochloric acid, and extracted with ethyl acetate. The organic layer was washed with brine, dehydrated over MgSO₄, and then concentrated. The residue was purified by silica gel column chromatography (methylene chloride) to obtain Compound I-2 (1.03 g, 66%).
¹H-NMR (400 MHz, CDCl₃): 8.06 (d, 2H, J=8.8Hz) 7.90 (br s, 1H), 7.75 (d, 2H, J=8.8Hz), 7.66 (d, 2H, J=1.5Hz), 7.64 (t, 1H, J=1.8Hz), 3.92 (s, 3H), 1.37 (s, 18H).

Compound I-2 (1.02 g, 2.78 mmol) was dissolved in THF(30 ml), and gradually added with DIBAL (8.34 ml, 1 M solution in toluene, 8.34 mmol) at -20°C. After 30 minutes, the reaction mixture was poured into 2 N hydrochloric acid and extracted with ethyl acetate. The organic layer was washed with brine and dehydrated over MgSO₄, and the solvent was concentrated. The residue was purified by silica gel column chromatography (ethyl acetate : n-hexane = 1:1) to obtain Compound I-3 (786 mg, 83%).
¹H-NMR (400 MHz, CDCl₃): 7.78 (br s, 1H), 7.67 (d, 2H, J=1.8Hz), 7.65 (d, 2H, J=8.8Hz), 7.62 (t, 1H, J=1.8Hz), 7.38 (d, 2H, J=8.5Hz), 4.69 (d, 2H, J=5.9Hz), 1.37 (s, 18H).

Compound I-3 (780 mg, 2.30 mmol) was dissolved in methanol-free methylene chloride (22 ml), and the solution was added with PCC (992 mg, 4.60 mmol) and stirred at room temperature for 2.5 hours. The reaction mixture was concentrated, and the residue was purified by silica gel column chromatography (ethyl acetate : n-hexane = 1:4) to obtain compounds I-4 (704 mg, 91%).
¹H-NMR (400 MHz, CDCl₃): 9.96 (s, 1H), 7.97 (br s, 1H), 7.92 (d, 2H, J=8.4Hz), 7.85 (d, 2H, J=8.4Hz), 7.67 (d, 2H, J=1.8Hz), 7.66 (t, 1H, J=1.8Hz), 1.38 (s, 18H).

Compound I-4 (150 mg, 0.45 mmol) and 2,4-thiazolidinedione (52 mg, 0.45 mmol) were suspended in anhydrous toluene (10 ml), and the suspension was added with a solution of piperidine (11 mg, 0.13 mmol) and acetic acid (8 mg, 0.13 mmol) dissolved in anhydrous toluene (1.4 ml), and then refluxed at 120°C for 3.5 hours. The reaction mixture was poured into ice water and extracted with ethyl acetate. The organic layer was washed with brine and dehydrated over MgSO₄, and the solvent was concentrated to obtain TZ151 (194 mg, 99%).
TZ151: Yellow powder (ethyl acetate/n-hexane); mp > 300°C; ¹H-NMR: (400 MHz, DMSO-d₆, 30°C): 10.43 (s, 1H), 7.93 (d, 2H, J=8.4Hz) and 7.75 (s, 1H), 7.74 (d, 2H, J=1.8Hz), 7.63 (m, 3H), 1.35 (s, 18H); Anal. Calcd. for C₂₅H₂₈N₂O₃S, C: 68.78%, H: 6.46 %, N: 6.42%; Found, C: 68.70%, H: 6.59%, N: 6.15%.

### Example 3: Synthesis of TZ153

TZ153 was synthesized by using 3,5-di-tert-butylbenzoic acid (I-1) and methyl m-aminobenzoate as starting materials according to the method of Example 2.
TZ153: Pale yellow powder (ethyl acetate/n-hexane); mp 252°C; ¹H-NMR (400 MHz, DMSO-d₆, 30°C): 10.36 (s, 1H), 8.16 (br s, 1H), 7.76 (m, 4H), 7.63 (t, 1H, J=1.8Hz), 7.52 (t, 1H, J=8.1Hz), 7.37 (d, 1H, J=8.0Hz), 1.35 (s, 18H); Anal. Calcd. for C₂₅H₂₈N₂O₃S, C: 68.78%, H: 6.46%, N: 6.42%; Found, C: 68.81%, H: 6.60%, N: 6.59%.

### Example 4: Synthesis of TZ155

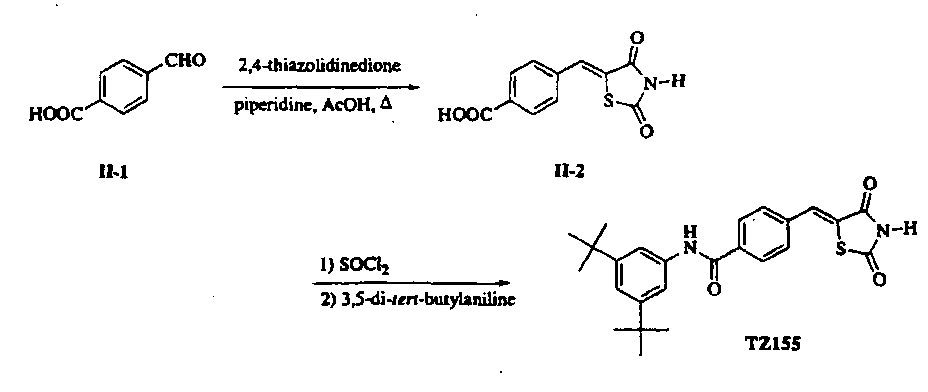

p-Formylbenzoic acid (II-1) (1.00 g, 6.67 mmol) and 2,4-thiazolidinedione (858 mg, 7.33 mmol) were suspended in anhydrous toluene (40 ml), and the mixture was added with a solution of piperidine (170 mg, 2.00 mmol) and acetic acid (120mg, 2.00 mmol) dissolved in anhydrous toluene (20 ml), and then refluxed at 120°C for 6 hours. The reaction mixture was cooled to room temperature and deposited crystals were collected by filtration. The crystals were washed with toluene, benzene, and aqueous 20% acetone solution, and dried to obtain Compound II-2 (1.57 g, 94%).
¹H-NMR (400 MHz, DMSO-d₆, 30°C): 8.04 (d, 2H, J=8.4Hz), 7.79 (s, 1H), 7.70 (d, 2H, J=8.4Hz).

Compound II-2 (250 mg, 1.00 mmol) was suspended in anhydrous benzene (12 ml), and the suspension was added with SOCl₂ (627 mg, 5.27 mmol) and refluxed for 14 hours. After the SOCl₂ was evaporated, the residue was suspended in anhydrous benzene (10 ml), and the mixture was added with 3,5-di-tert-butylaniline (210 mg, 1.00 mmol) and anhydrous pyridine (4 ml), and then stirred at room temperature for 2 hours. The reaction mixture was added with 2 N hydrochloric acid with floating ice and extracted with ethyl acetate. The organic layer was washed with brine, dehydrated over MgSO₄, and concentrated. The residue was purified by silica gel column chromatography (ethyl acetate : n-hexane = 3:2) to obtain TZ155 (390 mg, 89%).
TZ155: Pale yellow powder (ethyl acetate/n-hexane); mp 266-267°C; ¹H-NMR (400 MHz, DMSO-d₆, 30°C): 10.20 (s, 1H), 8.08 (d, 2H, J=8.4Hz), 7.87 (s, 1H), 7.74 (d, 2H, J=8.4Hz), 7.69 (d, 1H, J=1.5Hz), 7.16 (t, 1H, J=1.5Hz), 1.30 (s, 18H); Anal. Calcd. for C₂₅H₂₈N₂O₃S, C: 68.78%, H: 6.46%, N: 6.42%, Found, C: 68.52%, H: 6.52%, N: 6.37%.

### Example 5: Synthesis of TZ157

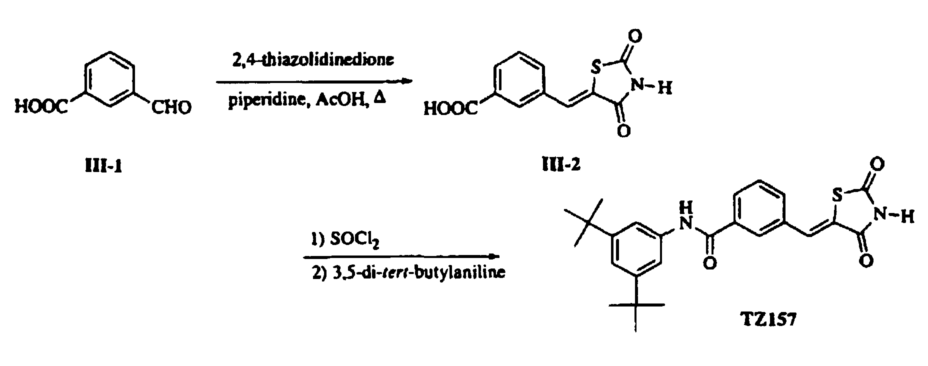

m-Formylbenzoate (III-1) (800 mg, 5.33mmol) and 2,4-thiazolidinedione (686 mg, 5.87 mmol) were suspended in anhydrous toluene (40 ml), and the suspension was added with a solution of piperidine (136 mg, 1.60 mmol) and acetic acid (96 mg, 1.60 mmol) dissolved in anhydrous toluene (16 ml), and then refluxed at 120°C for 4.5 hours. The reaction mixture was cooled to room temperature, and deposited crystals were collected by filtration. The crystals were washed with toluene, benzene, and aqueous 20% acetone solution, and dried to obtain Compound III-2 (1.017 g, 77%).
¹H-NMR (400 MHz, DMSO-d₆, 30°C): 8.14 (s, 1H), 8.01 (d, 1H, J=7.7Hz), 7.86 (s, 1H), 7.85 (d, 1H, J=7.7Hz), 7.66 (t, 1H, J=7.7Hz).

Compound III-2 (250 mg, 1.00 mmol) was suspended in anhydrous benzene (14 ml), and the suspension was added with SOCl₂ (627 mg, 5.27 mmol) and refluxed for 14 hours. After the SOCl₂ was evaporated, the residue was suspended in anhydrous benzene (10 ml). The mixture was added with 3,5-di-tert-butylaniline (210 mg, 1.00 mmol) and anhydrous pyridine (4 ml), and then stirred at room temperature for 2 hours. The reaction mixture was added with 2 N hydrochloric acid with floating ice and extracted with ethyl acetate. The organic layer was washed with brine, dehydrated over MgSO₄, and concentrated. The residue was purified by silica gel column chromatography (ethyl acetate : n-hexane = 3:4) to obtain TZ157 (292 mg, 67%).
TZ157: Colorless needles (ethyl acetate/n-hexane); mp 263°C; ¹H-NMR (400 MHz, DMSO-d₆, 30°C): 10.20 (s, 1H), 8.15 (s, 1H), 8.04 (d, 1H, J=7.7Hz), 7.87 (s, 1H), 7.78 (d, 1H, J=7.7Hz), 7.69 (t, 1H, J=7.7Hz), 7.67 (d, 2H, J=1.5Hz), 7.17 (t, 1H, J=1.5Hz), 1.30 (s, 18H); Anal. Calcd. for C₂₅H₂₈N₂O₃S, C: 68.78%, H: 6.46%, N: 6.42%, Found, C: 68.82%, H: 6.65%. N: 6.56%.

### Example 6: Synthesis of TZ161

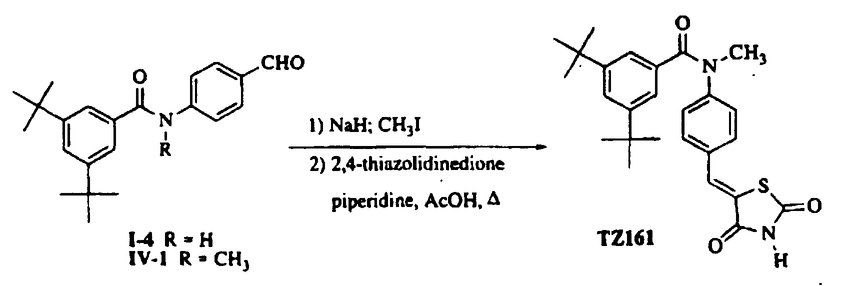

NaH (97.6 mg, 60%, 2.45 mmol) was washed with n-hexane and suspended in dimethylformamide (DMF, 1 ml). The suspension was added with the aldehyde I-4 (see, Example 2, 550 mg, 1.63 mmol) dissolved in DMF (10 ml) and stirred at room temperature for 20 minutes. The reaction mixture was added with methyl iodide (0.19 ml, 3.05 mmol) and stirred for 45 minutes. After DMF was evaporated, the residue was added with water and extracted with methylene chloride. The organic layer was washed with brine and dehydrated over MgSO₄, and then the solvent was concentrated. The residue was purified by silica gel column chromatography (ethyl acetate : n-hexane = 1:3) to obtain Compound IV-1 (389 mg, 68%).
¹H-NMR (400 MHz, CDCl₃): 9.90 (s, 1H), 7.73 (d, 2H, J=8.4Hz), 7.31 (t, 1H, J=1.8Hz), 7.31 (t, 1H, J=1.8Hz), 7.15 (d, 2H, J=8.4Hz), 7.13 (d, 2H, J=1.8Hz), 3.56 (s, 3H), 1.14 (s, 18H).

Compound IV-1 (385 mg, 1.10 mmol) and 2,4-thiazolidinedione (128 mg, 1.10 mmol) were suspended in anhydrous toluene (8 ml), and the suspension was added with a solution of piperidine (26 mg, 0.33 mmol) and acetic acid (20 mg, 0.33 mmol) dissolved in anhydrous toluene (3 ml), and then refluxed at 120°C for 1.5 hours. The reaction mixture was poured into ice water and extracted with ethyl acetate. The organic layer was washed with brine, dehydrated over MgSO₄, and the solvent was concentrated. The residue was purified by silica gel column chromatography (ethyl acetate : n-hexane = 1:1) to obtain TZ161 (417 mg, 84.5%).
TZ161: Yellow plate (ethyl acetate/n-hexane); mp 265°C; ¹H-NMR (400 MHz, DMSO-d₆, 30°C): 7.70 (s, 1H), 7.46 (d, 2H, J=8.4Hz), 7.29 (t, 1H, J=1.5Hz), 7.26 (d, 2H, J=8.4Hz), 7.09 (d, 2H, J=1.5Hz), 3.41 (s, 3H), 1.12 (s, 18H); Anal. Calcd. for C₂₆H₃₀N₂O₃S, C: 69.31%, H:6.71%, N: 6.22%, Found, C: 69.01%, H: 6.68%, N: 5.93%.

### Example 7: Synthesis of TZ163

TZ163 was synthesized by using 3-(3,5-di-tert-butylphenylcarbamoyl)benzaldehyde (synthesized from methyl m-aminobenzoate in the same manner as that of the preparation of Compound I-4) as a starting material according to the method of Example 6.
TZ163: Yellow plates (ethyl acetate/n-hexane); mp 195°C; ¹H-NMR (400 MHz, DMSO-d₆, 30°C): 7.61 (s, 1H), 7.46 (t, 1H, J=7.7Hz), 7.38 (m, 2H), 7.27 (t, 1H, J=1.8Hz), 7.14 (br s, 1H), 7.08 (d, 2H, J=1.8Hz), 3.42 (s, 3H), 1.11 (s, 18H); Anal. Calcd. for C₂₆H₃₀N₂O₃S, C: 69.31%, H: 6.71%, N: 6.22%, Found, C: 69.41%, H: 6.92%, N: 5.99%.

### Example 8: Synthesis of TZ165

By using the thiazolidine II-2 (see, Example 4) and 2,3,5-di-tert-butyl-N-methylaniline as starting materials, TZ165 was synthesized according to the method of Example 4 (79%).
TZ165: Pale yellow prisms (ethyl acetate/n-hexane); mp 253-254°C; ¹H-NMR (400 MHz, DMSO-d₆, 30°C): 7.67 (s, 1H), 7.38 (d, 2H, J=8.4Hz), 7.29 (d, 2H, J=8.4Hz), 7.11 (s, 1H), 6.93 (s, 2H), 3.42 (s, 3H), 1.12 (s, 18H); Anal. Calcd. for C₂₆H₃₀N₂O₃S, C: 69.31%, H: 6.71%, N: 6.22%, Found, C: 69.05%, H: 6.53%, N: 6.48%.

### Example 9: Synthesis of TZ167

By using the thiazolidine III-2 (see, Example 5) and 3,5-di-tert-butyl-N-methylaniline as starting materials, TZ167 was synthesized according to the method of Example 5 (76%).
TZ167: Colorless prisms (ethyl acetate/n-hexane); mp 238°C; ¹H-NMR (400 MHz, DMSO-d₆, 30°C): 7.58 (s, 1H), 7.48 (m, 2H), 7.23 (br s, 1H), 7.10 (s, 1H), 6.93 (d, 2H, J=1.5Hz), 3.44 (s, 3H), 1.11 (s, 18H); Anal. Calcd. for C₂₆H₃₀N₂O₃S, C: 69.31%, H: 6.71%, N: 6.22%, Found, C: 69.13%, H: 6.78%, N: 6.34%.

### Example 10: Synthesis of TZ175

By using 2,4-xylidine and the thiazolidine II-2 (see, Example 4) as starting materials, TZ175 was synthesized according to the method of Example 4 (88%).
TZ175: Pale pink powder (methylene chloride/methanol); mp 269°C; ¹H-NMR (400 MHz, DMSO-d₆, 30°C): 9.89 (s, 1H), 8.08 (d, 2H, J=8.4Hz), 7.86 (s, 1H), 7.73 (d, 2H, J=8.4Hz), 7.21 (d, 1H, J=8.1Hz), 7.08 (s, 1H), 7.02 (d, 1H, J=8.1Hz), 2.29 (s, 3H), 2.20 (s, 3H); Anal. Calcd. for C₁₉H₁₆N₂O₃S, C: 64.76%, H: 4.58%, N: 7.95% Found, C: 64.51%, H: 4.67%, N: 8.07%.

### Example 11: Synthesis of TZ177

By using 2,4-xylidine and the thiazolidine III-2 (see, Example 5) as starting materials, TZ177 was synthesized according to the method of Example 5 (31%).
TZ177: Colorless needles (methylene chloride/methanol); mp 245°C ¹H-NMR (400 MHz, DMSO-d₆, 30°C): 9.90 (s, 1H), 8.15 (s, 1H), 8.04 (d, 1H, J=7.7Hz), 7.87 (s, 1H), 7.79 (d, 1H, J=8.1Hz), 7.68 (t, 1H, J=7.7Hz), 7.23 (d, 1H, J=8.1Hz), 7.09 (s, 1H), 7.03 (d, 1H, J=8.1Hz), 2.29 (s, 3H), 2.21 (s, 3H); Anal. Calcd. for C₁₉H₁₆N₂O₃S, C: 64.76%, H: 4.58%, N: 7.95%; Found, C: 64.57%, H: 4.41%, N: 7.89%.

### Example 12: Synthesis of TZ181

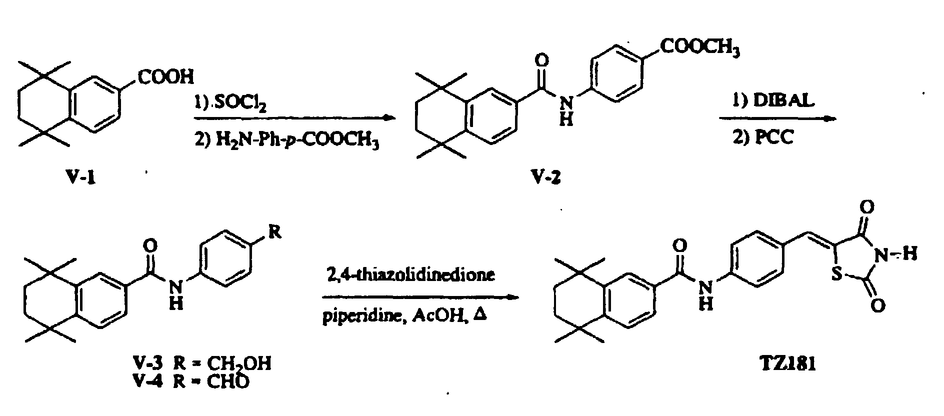

5,6,7,8-Tetrahydro-5,5,8,8-tetramethyl-2-naphthoic acid (V-1, 700 mg, 3.01 mmol) was suspended in thionyl chloride (8 ml), and the mixture was added with one drop of DMF, and then stirred at room temperature for 2 hours. The thionyl chloride was evaporated, and the residue was added with methyl p-aminobenzoate (450 mg, 2.98 mmol) and 4-dimethylaminopyridine (5 mg), and then the mixture was dissolved in anhydrous pyridine (20 ml) and stirred at room temperature overnight. The reaction mixture was poured into 2 N hydrochloric acid and extracted with ethyl acetate. The organic layer was washed with 2 N hydrochloric acid, water and brine, dehydrated over Na₂SO₄, and concentrated to obtain Compound V-2 (97%).

Compound V-2 (183 mg, 0.50 mmol) was dissolved in THF (10 ml), and the solution was gradually added with DIBAL (1.5 ml, 1 M solution in toluene, 1.5 mmol) at -45°C. After 30 minutes, the reaction mixture was poured into 2 N hydrochloric acid and extracted with ethyl acetate. The organic layer was washed with brine and dehydrated over Na₂SO₄, and the solvent was concentrated. The residue was purified by silica gel column chromatography (ethyl acetate : methylene chloride = 1:3) to obtain Compound V-3 (142 mg, 84%).
¹H-NMR (400 MHz, CDCl₃): 7.86 (d, 1H, J=2.2Hz), 7.78 (br s, 1H), 7.63 (d, 2H, J=8.4Hz), 7.55 (dd, 1H, J=2.0, 8.2Hz), 7.40 (d, 1H, J=8.8Hz), 7.37 (d, 2H, J=8.4Hz), 4.68 (s, 2H), 1.72 (s, 4H), 1.33 (s, 6H), 1.31 (s, 6H).

Compound V-3 (140 mg, 0.42 mmol) was dissolved in methanol-free methylene chloride (10 ml), and the solution was added with PCC (100 mg, 0.46 mmol) and stirred at room temperature for one hour. The reaction mixture was concentrated and purified by silica gel column chromatography (methylene chloride) to obtain Compound V-4 (99 mg, 71%).
¹H-NMR (400 MHz, CDCl₃): 9.95 (s, 1H) 7.92 (br s, 1H), 7.91 (d, 2H, J=8.8Hz), 7.87 (d, 1H, J=1.8Hz), 7.84 (d, 2H, J=8.8Hz), 7.56 (dd, 1H, J=2.0, 8.3Hz), 7.43 (d, 1H, J=8.4Hz), 1.73 (s, 4H), 1.34 (s, 6H), 1.32 (s, 6H).

Compound V-4 (73 mg, 0.22 mmol) and 2,4-thiazolidinedione (30 mg, 0.26 mmol) were suspended in anhydrous toluene (4 ml). Piperidine (173 µl) and acetic acid (100 µl) were dissolved in anhydrous toluene (25 ml), and the above suspension was added with the resulting solution (3 ml) and then refluxed at 120°C for 2 hours. The reaction mixture was poured into ice water and extracted with ethyl acetate. The organic layer was washed with 2 N hydrochloric acid and water, and dehydrated over Na₂SO₄. The solvent was concentrated to obtain TZ181 (100 mg, quantitative).
TZ181: Yellow needles (ethyl acetate/n-hexane); mp 288-290°C; ¹H-NMR (400 MHz, DMSO-d₆, 30°C): 12.52 (s, 1H) 10.36 (s, 1H), 7.94 (d, 2H, J=8.8Hz), 7.88 (d, 1H, J=2.2Hz), 7.76 (S, 1H), 7.71 (dd, 2H, J=2.2, 8.4Hz), 7.60 (d, 2H, J=8.8Hz), 7.48 (d, 1H, J=98.3Hz), 1.68 (s, 4H), 1.31 (s, 6H), 1.28 (S, 6H); Anal. Calcd. for C₂₅H₂₆N₂O₃S, C: 69.10%, H: 6.03 % N: 6.45%; Found, C: 69.05%, H: 6.23%, N: 6.55%.

### Example 13: Synthesis of TZ183

TZ183 was synthesized by using 5,6,7,8-tetrahydro-5,5,8,8-tetramethyl-2-naphthoic acid (V-1) and methyl m-aminobenzoate as starting materials according to the method of Example 12.
TZ183: Colorless powder (ethyl acetate/n-hexane); mp 183°C; ¹H-NMR (400 MHz, DMSO-d₆, 30°C): 10.29 (s, 1H), 8.15 (s, 1H), 7.88 (d, 1H, J=1.8Hz), 7.76 (d, 1H, J=1.8Hz), 7.26 (s, 1H), 7.26 (s, 1H), 6.71 (dd, 1H, J=8.4Hz, 1.8Hz), 6.50 (t, 1H, J=7.7Hz), 6.49 (d, 1H, J=8.1Hz), 6.35 (d, 1H, J=2.1Hz), 1.69 (s, 4H), 1.31 (s, 6H), 1.28 (s, 6H); Anal. Calcd. for C₂₅H₂₆N₂O₃S, C: 69.10%, H:6.03%, N: 6.45%; Found, C: 68.81%, H:5.92%, N: 6.51%.

### Example 14: Synthesis of TZ185

TZ185 was synthesized by using 5,6,7,8-tetrahydro-5,5,8,8-tetramethyl-2-naphthylamine and the thiazolidine II-2 (see, Example 4) as starting materials according to the method of Example 4.
TZ185: Pale orange plates (ethyl acetate/n-hexane); mp 234°C; ¹H-NMR (400 MHz, DMSO-d₆, 30°C): 10.18 (s, 1H), 8.07 (d, 2H, J=8.4Hz), 7.86 (s, 1H), 7.73 (d, 2H, J=8.4Hz), 7.68 (d, 1H, J=2.2Hz), 7.57 (dd, 1H, J=8.4Hz, 2.2Hz), 7.28 (d, 1H, J=8.4Hz), 1.65 (s, 4H), 1.25 (s, 6H), 1.24 (s, 6H); Anal. Calcd. for C₂₅H₂₆N₂O₃S, C: 69.10%, H:6.03%, N: 6.45%; Found, C: 69.40%, H: 6.10%, N: 6.55%.

### Example 15: Synthesis of TZ187

TZ187 was synthesized by using 5,6,7,8-tetrahydro-5,5,8,8-tetramethyl-2-naphthylamine and the thiazolidine III-2 (see, Example 5) as starting materials according to the method of Example 5.
TZ187: Colorless plates (ethyl acetate/n-hexane); mp 187°C; ¹H-NMR (40 MHz, DMSO-d₆, 30°C): 10.18 (s, 1H), 8.14 (s, 1H), 8.03 (d, 2H, J=7.7Hz), 7.87 (s, 1H), 7.78 (d, 1H, J=7.7Hz), 7.68 (t, 1H, J=7.7Hz), 7.68 (d, 1H, J=2.2Hz), 7.56 (dd, 1H, J=8.8Hz, 2.2Hz), 7.29 (d, 1H, J=8.4Hz), 1.65 (s, 4H), 1.26 (s, 6H), 1.24 (s, 6H); Anal. Calcd. for C₂₅H₂₆N₂O₃S, C: 69.10%, H: 6.03%, N:6.45%; Found, C: 68.81%, H: 6.21%, N: 6.37%.

### Example 16: Synthesis of TZ191

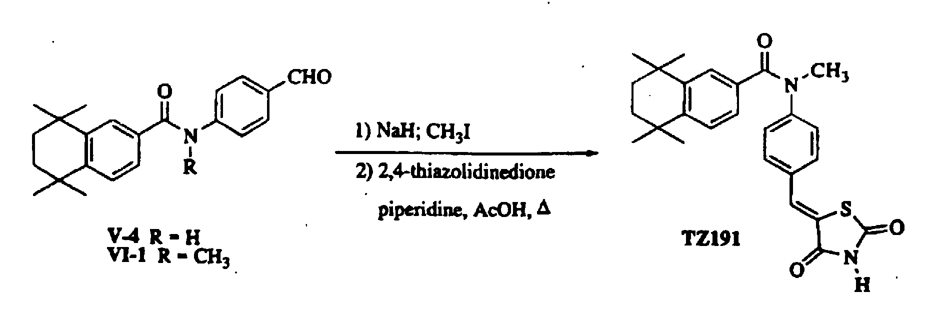

NaH (18 mg, 60%, 0.45 mmol) was washed with n-hexane, and suspended in DMF (1 ml). The suspension was added with the aldehyde V-4 (see, Example 12, 100 mg, 0.30 mmol) dissolved in DMF (4 ml) and stirred at room temperature for 15 minutes. The reaction mixture was added with methyl iodide (0.07 ml, 1.12 mmol) and stirred for 30 minutes. After the DMF was evaporated, the residue was added with water and extracted with methylene chloride. After the organic layer was washed with brine and dehydrated over MgSO₄, the solvent was concentrated. The residue was purified by silica gel column chromatography (ethyl acetate : n-hexane = 1:2) to obtain Compound VI-1 (388.9 mg, 63%).
¹H-NMR (400 MHz, CDCl₃): 9.92 (s, 1H), 7.75 (d, 2H, J=8.4Hz), 7.24 (dd, 1H, J=8.1, 1.8Hz), 7.19 (d, 1H, J=8.4Hz), 7.18 (d, 1H, J=8.4Hz), 7.04 (d, 1H, J=1.8Hz), 3.55 (s, 3H), 1.60 (m, 4H), 1.20 (s, 6H), 0.93 (s, 6H).

Compound VI-1 (60 mg, 0.17 mmol) and 2,4-thiazolidinedione (20 mg, 0.17 mmol) were suspended in anhydrous toluene (4 ml). The suspension was added with a solution of piperidine (4.4 mg, 0.052 mmol) and acetic acid (3.1 mg, 0.052 mmol) dissolved in anhydrous toluene (0.5 ml), and refluxed at 120°C for 40 minutes. The reaction mixture was poured into ice water and extracted with ethyl acetate. The organic layer was washed with brine and dehydrated over MgSO₄, and the solvent was concentrated. The residue was purified by silica gel column chromatography (ethyl acetate : n-hexane = 1:3) to obtain TZ191 (417 mg, 93%).
TZ191: Yellow powder (ethyl acetate/n-hexane): mp 235°C; ¹H-NMR (400 MHz, DMSO-d₆, 30°C): 7.71 (s, 1H), 7.48 (d, 2H, J=8.8Hz), 7.28 (d, 2H, J=8.4Hz), 7.27 (d, 1H, J=8.4Hz), 7.22 (dd, 1H, J=8.4, 1.5Hz), 6.98 (d, 1H, J=1.8Hz), 3.40 (s, 3H), 1.53 (m, 4H), 1.17 (s, 6H), 0.89 (s, 6H); Anal. Calcd. for C₂₆H₂₈N₂O₃S, C: 69.62% and H: 6.29% N: 6.24%, Found, C: 69.33%, H: 6.38%, N: 6.31%.

### Example 17: Synthesis of TZ193

TZ193 was synthesized by using 3-(5,6,7,8-tetrahydro-5,5,8,8-tetramethyl-2-naphthylcarbamoyl)benzaldehyde (synthesized from methyl m-aminobenzoate in the same manner as that of the synthesis of Compound V-4) as a starting material according to the method of Example 16.
TZ193: Colorless plates (ethyl acetate/n-hexane); mp 188°C; ¹H-NMR (400 MHz, DMSO-d₆, 30°C): 7.64 (s, 1H), 7.47 (t, 1H, J=7.7Hz), 7.38 (m, 2H), 7.24 (d, 1H, J=8.1Hz), 7.16 (dd, 1H, J=8.4, 1.8Hz), 7.03 (d, 1H, J=1.8Hz), 3.41 (s, 3H), 1.52 (s, 4H), 1.14 (s, 6H), 0.91 (s, 6H); Anal. Calcd. for C₂₆H₂₈N₂O₃S, C: 69.62%, H: 6.29%, N: 6.24%, Found, C: 69.65%, H: 6.16%, N: 6.08%.

### Example 18: Synthesis of TZ195

From the thiazolidine II-2 (see Example 4) and 5,6,7,8-tetrahydro-N,5,5,8,8-pentamethyl-2-naphthylamine, TZ195 was synthesized according to the method of Example 4 (80%).
TZ195: Pale yellow plates (ethyl acetate/n-hexane); mp 233°C; ¹H-NMR (400 MHz, DMSO-d₆, 30°C): 7.69 (s, 1H), 7.39 (d, 2H, J=8.1Hz), 7.31 (d, 2H, J=8.1Hz), 7.26 (d, 2H, J=8.8Hz), 7.06 (dd, 1H, J=8.4, 2.6Hz), 6.83 (br s, 1H), 3.37 (s, 3H), 1.50 (m, 4H), 1.16 (s, 6H), 0.91 (s, 6H); Anal. Calcd. for C₂₆H₂₈N₂O₃S, C: 69.62%, H: 6.29%, N: 6.24%, Found, C: 69.38%, H: 6.42%, N: 6.02%.

### Example 19: Synthesis of TZ197

From the thiazolidine III-2 (see Example 5) and 5,6,7,8-tetrahydro-N,5,5,8,8-pentamethyl-2-naphthylamine, TZ197 was synthesized according to the method of Example 5 (70%).
TZ197: Pale yellow prisms (ethyl acetate/n-hexane); mp 237°C; ¹H-NMR (400 MHz, DMSO-d₆, 30°C): 7.59 (s, 1H), 7.48 (d, 1H, J=7.0Hz), 7.42 (m, 2H), 7.24 (d, 1H, J=8.4Hz), 7.19 (s, 1H), 7.04 (dd, 1H, J=8.4, 2.2Hz), 6.85 (d, 1H, J=2.2Hz), 3.41 (s, 3H), 1.51 (s, 4H), 1.14 (s, 6H), 0.91 (s, 6H); Anal. Calcd. for C₂₆H₂₈N₂O₃S, C: 69.62%, H: 6.29%, N: 6.24%, Found, C: 69.51%, H: 6.37%, N: 6.27%.

### Example 20: Synthesis of TZ201

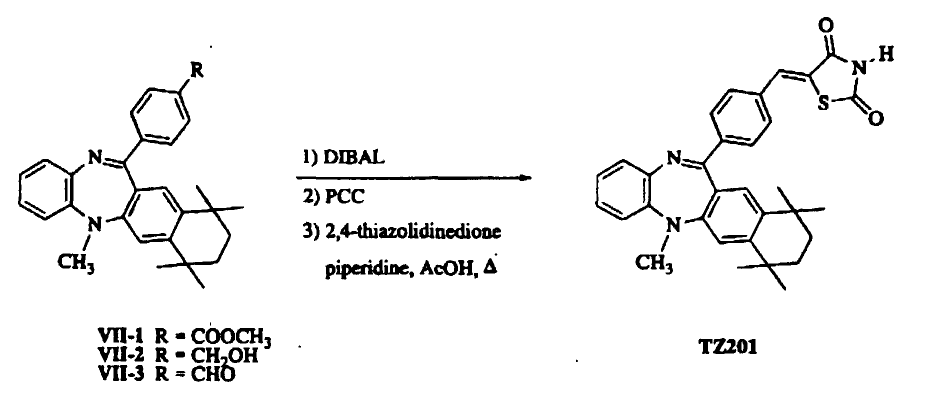

The ester compound VII-1 (110 mg, 0.24 mmol) was dissolved in THF (10 ml), and the solution was gradually added with DIBAL (1.5 ml) (1 M solution in toluene, 1.5 mmol) at -20°C. After 3 hours, the reaction mixture was poured into 2 N hydrochloric acid and extracted with ethyl acetate. The organic layer was washed with brine and dehydrated over Na₂SO₄, and the solvent was concentrated. The residue was purified by silica gel column chromatography (ethyl acetate : methylene chloride = 1:4) to obtain Compound VII-2 (100 mg, 97%).
¹H-NMR (400 MHz, CDCl₃): 7.81 (d, 2H, J= 8.4Hz), 7.40 (d, 2H, J=8.4Hz), 7.31 (d, 1H, J=7.3Hz), 7.13 (dt, 1H, J=1.8, 7.3Hz), 7.08 (dt, 1H, J=1.5, 7.3Hz), 6.97 (dd, 1H, J=1.5, 7.7Hz), 6.94 (s, 1H), 6.92 (s, 1H), 4.77 (d, 2H, J=4.4Hz), 3.25 (s, 3H), 1.64 (m, 4H), 1.32 (s, 3H), 1.26 (s, 3H), 1.14 (s, 3H), 1.05 (s, 3H).

Compound VII-2 (100 mg, 0.24mmol) was dissolved in methanol-free methylene chloride (10 ml), and the solution was added with PCC (60 mg, 0.28 mmol) and stirred at room temperature for one hour. The reaction mixture was concentrated and purified by silica gel column chromatography (ethyl acetate : methylene chloride =1:50) to obtain Compound VII-3 (72 mg, 72%).
¹H-NMR (400 MHz, CDCl₃): 10.10 (s, 1H), 7.98 (d, 2H, J=8.0Hz), 7.92 (d, 2H, J=8.8Hz), 7.32 (d, 1H, J=7.7Hz), 7.17 (dt, 1H, J=1.5, 8.0Hz), 7.10 (dt, 1H, J=1.5, 7.7Hz), 6.98 (dd, 1H, J=1.5, 8.1Hz), 6.93 (s, 1H), 6.86 (s, 1H), 3.26 (s, 3H), 1.65 (m, 4H), 1.32 (s, 3H), 1.27 (s, 3H), 1.12 (s, 3H), 1.04 (s, 3H).

Compound VII-3 (70 mg, 0.17 mmol) and 2,4-thiazolidinedione (20 mg, 0.17 mmol) were suspended in anhydrous toluene (4 ml). Piperidine (173 µl) and acetic acid (100 µl) were dissolved in anhydrous toluene (25 ml). The above suspension was added with the resulting solution (2.5 ml) and then refluxed at 120°C for 2 hours. The reaction mixture was poured into ice water and extracted with ethyl acetate. The organic layer was washed with 2 N hydrochloric acid and water, and dehydrated over Na₂SO₄, and the solvent was concentrated to obtain TZ201 (73 mg, 84%).
TZ201: Red needles (ethyl acetate/methanol); mp >300°C; ¹H-NMR (400 MHz, DMSO-d₆, 30°C): 12.62 (s, 1H), 7.83 (s, 1H), 7.82 (d, 2H, J=8.7Hz), 7.69 (d, 2H, J=8.3Hz), 7.16-7.22 (m, 2H), 7.09 (m, 2H), 7.06 (s, 1H), 6.90 (s, 1H), 3.21 (s, 3H), 1.62 (m, 4H), 1.30 (s, 3H), 1.26 (s, 3H), 1.13 (s, 3H), 1.03 (S, 3H); Anal. Calcd. for C₃₂H₃₁N₃O₂S-H₂O, C: 71.23%, H: 6.16%, N: 7.79%; Found, C: 71.12%, H: 6.02%, N:7.71%.

### Example 21: Synthesis of TZ221

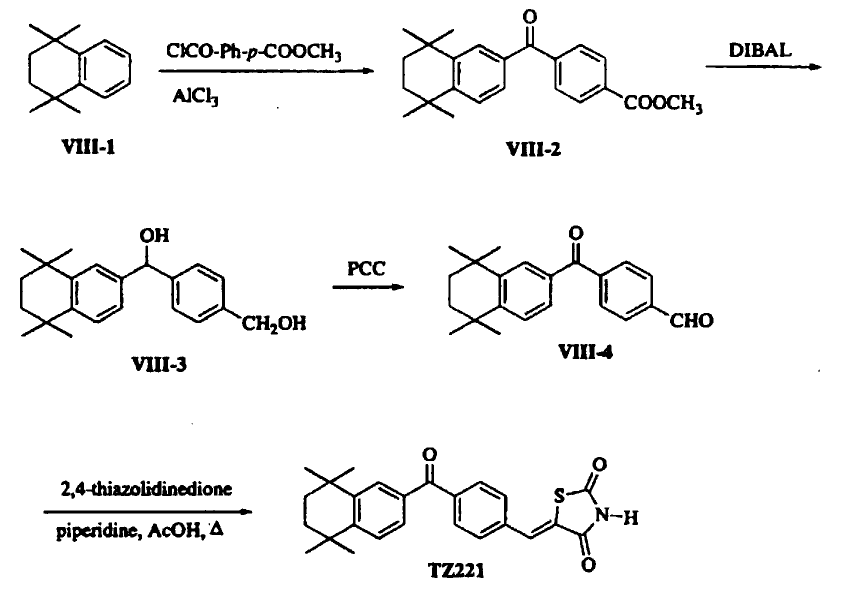

1,2,3,4-Tetrahydro-1,1,4,4-tetramethylnaphthalene (VIII- 1, 1.00g, 5.32 mmol) and terephthalic acid monomethyl ester chloride (1.06 g, 5.32 mmol) were dissolved in methanol-free methylene chlorides (20 ml), and the solution was added with aluminium chloride (1.42 g, 10.64 mmol) with ice cooling, and then refluxed for 30 minutes. The reaction mixture was poured into ice water and extracted with ethyl acetate. The organic layer was washed with water and brine, dehydrated over MgSO₄, and concentrated. The residue was purified by silica gel column chromatography (ethyl acetate : hexane = 1:20, then 1:10) to obtain Compound VIII-2 (1.30 g, 70%).
¹H-NMR (400 MHz, CDCl₃): 8.14 (d, 2H, J=8.4Hz), 7.83 (d, 2H, J=8.4Hz), 7.78 (d, 1H, J=1.8Hz), 7.53 (dd, 1H, J=8.4, 1.8Hz), 7.40 (d, 1H, J=8.0Hz), 3.97 (s, 3H), 1.72 (s, 4H), 1.32 (s, 6H), 1.29 (s, 6H).

Compound VIII-2 (1.20 g, 3.43 mmol) was dissolved in THF (15 ml), and gradually added with DIBAL (13.7ml, 1 M solution in toluene, 13.7 mmol) dropwise while stirring was continued at -78°C under argon atmosphere. After one hour, the reaction mixture was poured into 1 N hydrochloric acid and extracted with ethyl acetate. The organic layer was washed with brine, dehydrated over MgSO₄, and concentrated. The residue was purified by silica gel column chromatography (ethyl acetate : hexane = 1:3). The resulting product was found to be obtained by reduction of only the ketone group (937.5 mg). Accordingly, the resulting product was further reduced by DIBAL at 0°C for 30 minutes and subjected to the same post-treatment to obtain Compound VIII-3 (896 mg, 81%).
¹H-NMR (400 MHz, CDCl₃): 7.40 (d, 2H, J=8.1Hz), 7.34 (m, 3H), 7.25 (d, 1H, J=8.0Hz), 7.05 (dd, 1H, J=8.0, 1.8Hz), 5.80 (s, 1H), 4.68 (s, 2H), 2.15 (br s, 1H), 1.67 (s, 4H), 1.26 (s, 6H), 1.25 (s, 6H).

Alumina (4.70 g) and PCC (2.65 g, 12.3 mmol) were suspended in methanol-free methylene chloride (10 ml) under argon atmosphere, and the suspension was gradually added with Compound VIII-3 (810 mg, 2.50 mmol) dissolved in methanol-free methylene chloride (10 ml). After one hour, the reaction mixture was concentrated and purified by silica gel column chromatography (ethyl acetate : n-hexane = 1:7) to obtain Compound VIII-4 (798 mg, 99.7%).
¹H-NMR (400 MHz, CDCl₃): 10.14 (s, 1H), 8.00 (d, 2H, J=8.4Hz), 7.91 (d, 2H, J=8.1Hz), 7.80 (d, 1H, J=1.8Hz), 7.53 (dd, 1H, J=8.4, 2.2Hz), 7.41 (d, 1H, J=8.1Hz), 1.73 (s, 4H), 1.32 (s, 6H), 1.30 (s, 6H).

Compound VIII-4 (790 mg, 2.47 mmol) and 2,4-thiazolidinedione (319 mg, 2.72 mmol) were suspended in anhydrous toluene (20 ml), and the suspension was added with a solution of piperidine (63 mg, 0.74 mmol) and acetic acid (45 mg, 0.74 mmol) dissolved in anhydrous toluene (8 ml), and refluxed at 120°C for 3 hours. The reaction mixture was poured into ice water and extracted with ethyl acetate. The organic layer was washed with brine, dehydrated over MgSO₄, and then concentrated. The residue was purified by silica gel column chromatography (ethyl acetate : hexane = 1:2) to obtain TZ221 (328 mg, 32%).
TZ221: Colorless powder (ethyl acetate/n-hexane); mp 204°C; ¹H-NMR (400 MHz, CDCl₃): 8.46 (s, 1H), 7.90 (d, 2H, J=8.4Hz), 7.80 (d, 1H, J=1.8Hz), 7.60 (d, 2H, J=8.1Hz), 7.53 (d, 1H, J=8.0, 1.8Hz), 7.41 (d, 1H, J=8.1Hz), 1.73 (s, 4H), 1.33 (s, 6H), 1.31 (s, 6H); Anal. Calcd. for C₂₅H₂₅NO₃S, C, 71.57; H, 6.01%; N, 3.34%; Found, C, 71.28%; H, 5.92%; N, 3.09%.

### Example 22: Synthesis of TZ223

TZ223 was synthesized by using 1,2,3,4-tetrahydro-1,1,4,4-tetramethylnaphthalene (VIII-1) and isophthalic acid monomethyl ester chloride as starting materials according to the method of Example 21.
TZ223; Yellow prisms (ethyl acetate/n-hexane); mp 189°C; ¹H-NMR (400 MHz, CDCl₃): 8.46 (br s, 1H), 7.91 (s, 1H), 7.90 (s, 1H), 7.86 (d, 1H, J=7.7Hz), 7.81 (d, 1H, J=1.8Hz), 7.70 (d, 1H, J=7.7Hz), 7.61 (t, 1H, J=7.7Hz), 7.52 (dd, 1H, J=8.1, 1.8Hz), 7.42 (d, 1H, J=8.1Hz), 1.73 (s, 4H), 1.33 (s, 6H), 1.31 (s, 6H); Anal. Calcd. for C₂₅H₂₅NO₃S, C: 71.57%, H: 6.01%, N: 3.34%; Found, C: 71.64%, H: 6.16%, N: 3.19%.

### Example 23: Synthesis of TZ225

TZ225 was synthesized by using 1,2,3,4-tetrahydro-1,1,4,4,6-pentamethylnaphthalene and terephthalic acid monomethyl ester chloride as starting materials according to the method of Example 21.
TZ225: Yellow prisms (ethyl acetate/n-hexane); mp 245°C; ¹H-NMR (400 MHz, CDCl₃): 8.67 (s, 1H), 7.91 (d, 1H, J=8.4Hz), 7.90 (s, 1H), 7.58 (d, 1H, J=8.8Hz), 7.26 (s, 1H), 7.21 (s, 1H), 2.33 (s, 3H), 1.70 (s, 4H), 1.32 (s, 6H), 1.22 (s, 6H); Anal. Calcd.for C₂₆H₂₇NO₃S, C: 72.03%, H: 6.28 % N: 3.23%; Found, C: 71.87%, H: 6.35%, N: 3.14%.

### Example 24: Synthesis of TZ227

TZ227 was synthesized by using 1,2,3,4-tetrahydro-1,1,4,4,6-pentamethylnaphthalene and isophthalic acid monomethyl ester chloride as starting materials according to the method of Example 21.
TZ227: Pale yellow prisms (ethyl acetate/n-hexane); mp 191°C; ¹H-NMR (400 MHz, CDCl₃): 8.40 (s, 1H), 7.87-7.92 (m, 2H), 7.86 (s, 1H), 7.69 (d, 1H, J=7.7Hz), 7.59 (t, 1H, J=7.7Hz), 7.25 (s, 1H), 7.23 (s, 1H), 2.32 (s, 3H), 1.71 (s, 4H), 1.33 (s, 6H), 1.22 (s, 6H); Anal. Calcd. for C₂₆H₂₇NO₃S, C: 72.03%, H: 6.28%. N: 3.23%; Found, C: 72.21%, H: 6.37%, N: 2.96%.

### Example 25: Synthesis of TZ241

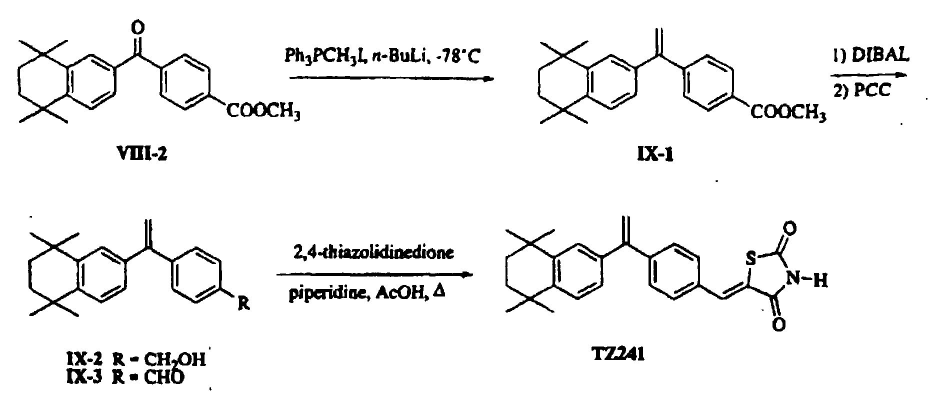

Ph₃PCH₃I (4.04 g, 10.1 mmol) was suspended in THF (5 ml), and the suspension was added with n-butyl lithium (8.36 ml, 13.4 mmol) at -78°C and stirred for 15 minutes. The suspension was added with Compound VIII-2 (2.35 g, 6.71 mmol) dissolved in THF (12 ml) and then stirred for one hour. The reaction mixture was added with water and extracted with methylene chloride. The organic layer was dehydrated over MgSO₄, concentrated and purified by silica gel column chromatography (ethyl acetate : n-hexane = 1:12.5) to obtain Compound IX-1 (680 mg, 30%).
¹H-NMR (400 MHz, CDCl₃): 8.00 (d, 2H, J=8.6Hz), 7.43 (d, 2H, J=8.4Hz), 7.26 (d, 1H, J=8.1Hz), 7.22 (d, 1H, J=1.8Hz), 7.07 (dd, 1H, J=8.3, 2.2Hz), 5.53 (d, 1H, J=1.1Hz), 5.47 (d, 1H, J=1.1Hz), 3.93 (s, 3H), 1.69 (s, 4H), 1.30 (s, 6H), 1.23 (s, 6H).

Compound IX-1 (675 mg, 2.01 mmol) was dissolved in THF (5 ml), and the solution was gradually added with DIBAL (6.0 ml, 1 M solution in toluene, 6.0 mmol) at -78°C, and then stirred at 0°C for 30 minutes. The reaction mixture was poured into 1 N hydrochloric acid and extracted with ethyl acetate. The organic layer was washed with brine, dehydrated over MgSO₄, and then concentrated. The residue was purified by silica gel column chromatography (ethyl acetate : n-hexane = 1:3) to obtain Compound IX-2 (619 mg, quantitative).
¹H-NMR (400 MHz, CDCl₃): 7.35 (m, 4H), 7.27 (d, 1H, J=1.8Hz), 7.25 (d, 1H, J=8.4Hz), 7.08 (dd, 1H, J=8.4, 2.2Hz), 5.44 (d, 1H, J=1.5Hz), 5.40 (d, 1H, J=1.1Hz), 4.72 (s, 2H), 1.69 (s, 4H), 1.29 (s, 6H), 1.24 (s, 6H).

Compound IX-2 (620 mg, 2.01 mmol) was dissolved in methanol-free methylene chloride (10 ml), and the solution was added with PCC (866 mg, 4.02 mmol) and stirred at room temperature for 1.5 hours. The reaction mixture was concentrated and the residue was purified by silica gel column chromatography (ethyl acetate : n-hexane = 1:8) to obtain Compound IX-3 (428.5 mg, 70%).
¹H-NMR (400 MHz, CDCl₃): 10.03 (s, 1H), 7.85 (d, 2H, J=8.4Hz), 7.53 (d, 2H, J=8.4Hz), 7.27 (d, 1H, J=8.1Hz), 7.23 (d, 1H, J=1.8Hz), 7.06 (dd, 1H, J=8.1, 1.8Hz), 5.57 (d, 1H, J=1.1Hz), 5.51 (d, 1H, J=0.7Hz), 1.70 (s, 4H), 1.30 (s, 6H), 1.24 (s, 6H).

Compound XII-4 (420 mg, 1.37 mmol) and 2,4-thiazolidinedione (162 mg, 1.38 mmol) was suspended in anhydrous toluene (8 ml), and the suspension was added with a solution of piperidine (32 mg, 0.38 mmol) and acetic acid (23 mg, 0.38 mmol) dissolved in anhydrous toluene (4 ml), and refluxed at 120°C for 2 hours. The reaction mixture was poured into ice water and extracted with ethyl acetate. The organic layer was washed with brine, dehydrated over MgSO₄, and then concentrated. The residue was purified by silica gel column chromatography (ethyl acetate : n-hexane = 1:4) to obtain TZ241 (449.2 mg, 81%).
TZ241: Pale yellow needles (ethyl acetate/n-hexane); mp 198°C; ¹H-NMR (400 MHz, CDCl₃): 8.42 (s, 1H), 7.88 (s, 1H), 7.48 (m, 4H), 7.27 (d, 1H, J=8.4Hz), 7.24 (d, 1H, J=1.8Hz), 7.06 (dd, 1H, J=8.4, 1.8Hz), 5.52 (s, 1H), 5.51 (s, 1H), 1.70 (s, 4H), 1.30 (s, 6H), 1.25 (s, 6H); Anal. Calcd. for C₂₆H₂₇NO₂S, C: 74.79%, H: 6.52%, N: 3.35%; Found, C: 74.59%, H: 6.51%. N: 3.32%.

### Example 26: Synthesis of TZ243

TZ243 was synthesized by using methyl m-(5,6,7,8-tetrahydro-5,5,8,8-tetramethyl-2-naphthoyl)benzoate as a starting material according to the method of Example 25.
TZ243: Colorless powder (ethyl acetate/n-hexane); mp 168°C; ¹H-NMR (400 MHz, CDCl₃): 8.30 (br s, 1H), 7.85 (s, 1H), 7.46 (m, 4H), 7.28 (d, 1H, J=8.1Hz), 7.25 (d, 1H, J=2.2Hz), 7.05 (dd, 1H, J=8.1Hz, 2.2Hz), 5.51 (d, 1H, J= 0.7Hz), 5.46 (d, 1H, J=1.1Hz), 1.70 (s, 4H), 1.33 (s, 6H), 1.25 (s, 6H); Anal. Calcd. for C₂₆H₂₇NO₂S - 1/4H₂O, C: 74.00%, H: 6.57%, N: 3.32%; Found, C: 74.00%, H: 6.60%, N: 3.36%.

### Example 27: Synthesis of TZ245

Ph₃PCH₃I (1.09 g, 2.70 mmol) was suspended in THF (5 ml), and the suspension was added with n-butyl lithium (2.22 ml, 3.56 mmol) at -78°C, and stirred for 15 minutes. The suspension was added with TZ225 (see Example 23, 800 mg, 1.78 mmol) dissolved in THF (6 ml), and stirred for one hour. The reaction mixture was added with water and extracted with methylene chloride. The organic layer was dehydrated over MgSO₄, and concentrated, and the residue was purified by silica gel column chromatography (ethyl acetate : n-hexane = 1:3) to obtain TZ245 (52 mg, 6.5%).
TZ245: Pale yellow powder (ethyl acetate/n-hexane); mp 281°C; ¹H-NMR (400 MHz, CDCl₃): 8.29 (s, 1H), 7.84 (s, 1H), 7.42 (m, 4H), 7.12 (s, 1H), 7.09 (s, 1H), 5.83 (d, 1H, J=1.1Hz), 5.32 (d, 1H, J=1.1Hz), 1.96 (s, 3H), 1.70 (s, 4H), 1.31 (s, 6H), 1.28 (s, 6H); Anal. Calcd. for C₂₇H₂₉NO₂S, C: 75.14%, H: 6.77 %, N: 3.25%; Found, C: 74.86%, H:6.81%, N: 3.33%.

### Example 28: Synthesis of TZ247

TZ247 was synthesized by using methyl m-(5,6,7,8-tetrahydro-3,5,5,8,8-pentamethyl-2-naphthoyl)benzoate as a starting material according to the method of Example 25.
TZ247: Pale yellow powder (ethyl acetate/n-hexane); mp 185°C; ¹H-NMR (400 MHz, CDCl₃): 8.19 (br s, 1H), 7.79 (s, 1H), 7.49 (d, 1H, J=7.7Hz), 7.43 (t, 1H, J=7.7Hz), 7.37 (d, 1H, J=7.7Hz), 7.25 (s, 1H), 7.13 (s, 1H), 7.12 (s, 1H), 5.80 (d, 1H, J=1.1Hz), 5.31 (d, 1H, J=1.1Hz), 1.96 (s, 3H), 1.72 (s, 4H), 1.32 (s, 6H), 1.29 (s, 6H); Anal. Calcd. for C₂₇H₂₉NO₂S, C: 75.14%, H: 6.77%, N: 3.25%; Found, C: 74.85%, H: 6.72%, N: 2.98%.

### Example 29: Synthesis of TZ315

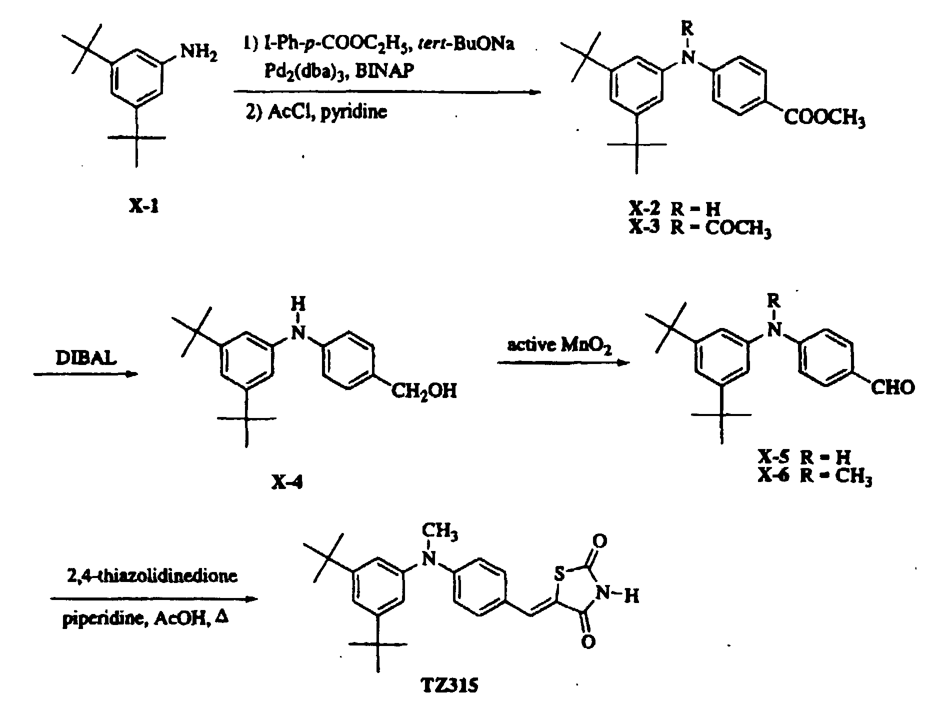

3,5-Di-tert-butylaniline (X-1, 1.00 g, 4.88 mmol), ethyl 4-iodobenzoate (1.37 g, 4.95 mmol), and tert-BuONa (549 mg, 5.68 mmol) were dissolved in anhydrous toluene (15 ml), and the solution was added with tris(dibenzylideneacetone)dipalladium(0) (91 mg) and (R)-BINAP (139 mg, 0.22 mmol) under argon atmosphere, and stirred at 100°C for one hour. The reaction mixture was cooled to room temperature, and extracted with ether. The organic layer was washed with brine, dehydrated over MgSO₄, and then concentrated. The residue was purified by silica gel column chromatography (ethyl acetate : n-hexane = 1:6) to obtain Compound X-2 (0.94 g, 55%).
¹H-NMR (400 MHz, CDCl₃): 7.92 (d, 2H, J=8.8Hz), 7.14 (t, 1H, J=1.8Hz), 7.02 (d, 2H, J=1.8Hz), 6.96 (d, 2H, J=8.8Hz), 4.33 (q, 2H, J=7.3Hz), 1.37 (t, 3H, J=7.3Hz), 1.32 (s, 18H).

Compound X-2 (935 mg, 2.65 mmol) was dissolved in anhydrous benzene (10 ml), and the solution was added with acetyl chloride (249 mg, 3.18 mmol) and anhydrous pyridine (0.5 ml), and then stirred at room temperature for 5 hours. The reaction mixture was added with ice water and extracted with ethyl acetate. The organic layer was washed with diluted hydrochloric acid and brine, dehydrated over MgSO₄, and concentrated. The residue was purified by silica gel column chromatography (ethyl acetate : n-hexane = 1:4) to obtain Compound X-3 (956 mg, 92%).
¹H-NMR (400 MHz, CDCl₃): 7.99 (d, 2H, J=8.4Hz), 7.39 (s, 1H), 7.34 (d, 2H, J=8.8Hz), 7.05 (d, 2H, J=1.8Hz), 4.35 (q, 2H, J=7.3Hz), 2.04 (s, 3H), 1.37 (t, 1H, J=7.0Hz), 1.30 (s, 18H).

Compound X-3 (950 mg, 2.40 mmol) was dissolved in THF (8 ml) under argon substitution, and the solution was gradually added dropwise with DIBAL (7.2 ml, 1 M solution in toluene, 7.20 mmol) with stirring at -78°C. After 15 minutes, the reaction mixture was poured into 2 N hydrochloric acid and extracted with ethyl acetate. The organic layer was washed with brine, dehydrated over MgSO₄, and then concentrated. The residue was purified by silica gel column chromatography (ethyl acetate : n-hexane = 1:2) to obtain Compound X-4 (412 mg, 55%).
¹H-NMR (400 MHz, CDCl₃): 7.27 (m, 3H), 7.04 (m, 3H), 6.96 (d, 2H, J=1.5Hz), 4.61 (s, 2H), 1.31 (s, 18H).

Compound X-4 (400 mg, 1.29 mmol) was dissolved in methanol-free methylene chloride (8 ml), and the solution was added with active MnO₂ (1.32 g, 85%, 12.9 mmol), and stirred at room temperature for 12 hours. The reaction mixture was filtered, and the filtrate was concentrated and purified by silica gel column chromatography (ethyl acetate : n-hexane = 1:8, then 1:4) to obtain Compound X-5 (184 mg, 46%).
¹H-NMR (400 MHz, CDCl₃), 9.78 (s, 1H), 7.74 (d, 2H, J=8.8Hz), 7.20 (t, 1H, J=1.8Hz), 7.05 (d, 1H, J=1.8Hz), 6.99 (d, 2H, J=8.4Hz), 6.17 (s, 1H), 1.33 (s, 18H).

NaH (34 mg, 60%, 0.87 mmol) was washed with n-hexane, and suspended in DMF (1 ml). The suspension was added with Compound X-5 (180 mg, 0.58 mmol) dissolved in DMF (5 ml) and stirred at room temperature for 15 minutes. The reaction mixture was added with CH₃I (0.14 ml, 2.25 mmol), and further stirred for one hour. After DMF was evaporated, the residue was added with water and extracted with methylene chloride. The organic layer was washed with brine, dehydrated over MgSO₄, and concentrated. Then, the residue was purified by silica gel column chromatography (ethyl acetate : n-hexane = 1:6) to obtain Compounds X-6 (173 mg, 92%).
¹H-NMR (400 MHz, CDCl₃): 9.75 (s, 1H), 7.68 (d, 2H, J=8.8Hz), 7.33 (t, 1H, J=1.8Hz), 7.05 (d, 2H, J=1.8Hz), 6.74 (d, 2H, J=8.8Hz), 3.40 (s, 3H), 1.33 (s, 18H).

Compound X-6 (170 mg, 0.53 mmol) and 2,4-thiazolidinedione (62 mg, 0.53 mmol) were suspended in anhydrous toluene (4 ml), and the suspension was added with a solution of piperidine (13.4 mg, 0.16 mmol) and acetic acid (9.5 mg, 0.16 mmol) dissolved in anhydrous toluene (1.6 ml), and refluxed at 120°C for 1.5 hours. The reaction mixture was pound into ice water and extracted with ethyl acetate. The organic layer was washed with brine, dehydrated over MgSO₄, and then concentrated. Then, the residue was purified by silica gel column chromatography (ethyl acetate : n-hexane = 1:3) to obtain TZ315 (197 mg, 89%).
TZ315: Yellow needles (ethyl acetate/n-hexane); mp 254°C; ¹H-NMR (400 MHz, CDCl₃): 8.11 (br s, 1H), 7.77 (s, 1H), 7.34 (m, 3H), 7.04 (d, 1H, J=1.8Hz), 6.77 (d, 2H, J=8.8Hz), 3.39 (s, 3H), 1.33 (s, 18H); Anal. Calcd. for C₂₅H₃₀N₂O₂S, C: 71.06%, H: 7.16%, N: 6.63%; Found, C: 70.96%, H: 7.17%, N: 6.81%.

### Example 30: Synthesis of TZ317

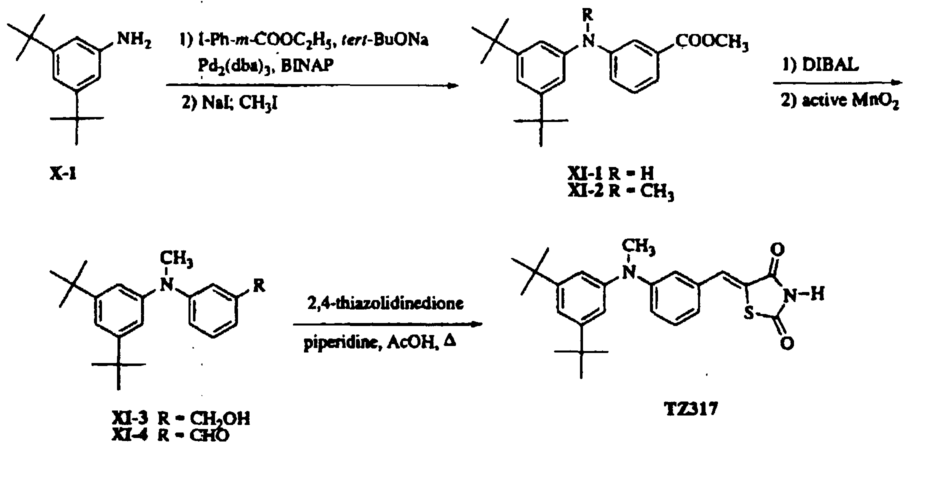

Methyl 3-iodobenzoate (1.37 g, 5.23 mmol), 3,5-di-tert-butylaniline (X-1) (1.00 g, 4.88 mmol), and tert-BuONa (549 mg, 5.68 mmol) were dissolved in anhydrous toluene (15 ml), and the solution was added with tris(dibenzylideneacetone)dipalladium(0) (91 mg) and (R)-BINAP (139 mg, 0.22 mmol) under argon substitution, and stirred at 80°C for one hour. The reaction mixture was cooled to room temperature, and extracted with ether. The organic layer was washed with brine, dehydrated over MgSO₄, and concentrated. The residue was then purified by silica gel column chromatography (ethyl acetate : n-hexane = 1:8) to obtain Compound XI-1 (crude product, 514 mg, 31%).
¹H-NMR (400 MHz, CDCl₃): 7.87 (d, 1H, J=7.7Hz), 7.75 (m, 1H), 7.53 (m, 1H), 7.29 (t, 1H, J=7.7Hz), 7.07 (t, 1H, J=1.5Hz), 6.98 (d, 2H, J=1.5Hz), 3.88 (s, 3H), 1.32 (s, 18H).

NaH (88 mg, 60%, 2.21 mmol) was washed with n-hexane, and suspended in DMF (1 ml). The suspension was added with Compound XI-1 (crude product, 500 mg, 1.47 mmol) dissolved in DMF (8 ml), and stirred at room temperature for 15 minutes. The reaction mixture was added with methyl iodide (0.35 ml, 5.62 mmol) and stirred for 3 hours. After DMF was evaporated, the residue was added with water and extracted with methylene chloride. The organic layer was washed with brine and dehydrated over MgSO₄, and the solvent was evaporated. The residue was purified by silica gel column chromatography (ethyl acetate : n-hexane = 1:10) to obtain Compound XI-2 (180 mg, 34.5%).
¹H-NMR (400 MHz, CDCl₃): 7.60 (m, 1H), 7.47 (d, 1H, J=7.7Hz), 7.23 (t, 1H, J=8.0Hz), 7.17 (t, 1H, J=1.8Hz), 7.04 (m, 1H), 6.99 (d, 2H, J=1.8Hz), 3.92 (s, 3H), 3.88 (s, 3H), 1.30 (s, 18H).

Compound XI-2 (170 mg, 0.48 mmol) was dissolved in THF (4 ml) under argon substitution, and gradually added dropwise with DIBAL (1.44 ml, 1 M solution in toluene, 1.44 mmol) with stirring at -78°C. After 30 minutes, the reaction mixture was poured into 2 N hydrochloric acid and extracted with ethyl acetate. The organic layer was washed with brine and dehydrated over MgSO₄, and the solvent was concentrated. The residue was purified by silica gel column chromatography (ethyl acetate : n-hexane = 1:3) to obtain Compound XI-3 (130 mg, 83%).
¹H-NMR (400 MHz, CDCl₃): 7.20 (t, 1H, J=1.8Hz), 7.14 (t, 1H, J=1.8Hz), 6.98 (d, 2H, J=1.8Hz), 6.92 (s, 1H), 6.81 (d, 2H, J=8.1Hz), 4.62 (d, 2H, J=5.9Hz), 3.34 (s, 3H), 1.30 (s, 18H).

Compound XI-3 (125 mg, 0.38 mmol) was dissolved in methanol-free methylene chloride (4 ml), and the solution was added with active MnO₂ (394 mg, 85%, 3.85 mmol) and stirred at room temperature for 6.5 hours. The reaction mixture was filtered, and the filtrate was concentrated. Then, the residue was purified by silica gel column chromatography (ethyl acetate : n-hexane = 1:6) to obtain Compound XI-4 (43.5 mg, 35%, 51 mg of XI-3 was collected).
¹H-NMR (400 MHz, CDCl₃): 9.92 (s, 1H), 7.35 (m, 1H), 7.32 (t, 1H, J=7.7Hz), 7.27 (m, 1H), 7.23 (t, 1H, J=1.8Hz), 7.07 (m, 1H), 7.02 (d, 1H, J=1.8Hz), 3.37 (s, 3H), 1.31 (s, 18H).

Compound XI-4 (65 mg, 0.20 mmol) and 2,4-thiazolidinedione (23 mg, 0.20 mmol) were suspended in anhydrous toluene (3 ml), and the suspension was added with a solution of piperidine (5.1 mg, 0.060 mmol) and acetic acid (3.6 mg, 0.060 mmol) dissolved in anhydrous toluene (0.6 ml), and refluxed at 120°C for 3.5 hours. The reaction mixture was poured into ice water and extracted with ethyl acetate. The organic layer was washed with brine and dehydrated over MgSO₄, and then the solvent was concentrated. The residue was purified by silica gel column chromatography (ethyl acetate : n-hexane = 1:2) to obtain TZ317 (88 mg, quantitative).
TZ317: Yellow needles (ethyl acetate/n-hexane); mp 234°C; ¹H-NMR (400 MHz, CDCl₃): 8.41 (br s, 1H), 7.77 (s, 1H), 7.22-7.57 (m, 2H), 7.01 (d, 2H, J=1.5Hz), 6.89 (dd, 2H, J=8.1, 2.2Hz), 6.83 (t, 1H, J=1.6Hz), 3.35 (s, 3H), 1.32 (s, 18H); Anal. Calcd. for C₂₅H₃₀N₂O₂S, C: 71.06%, H: 7.16%, N: 6.63%; Found, C: 70.88%, H: 7.09%, N: 6.36%.

### Example 31: Synthesis of TZ321

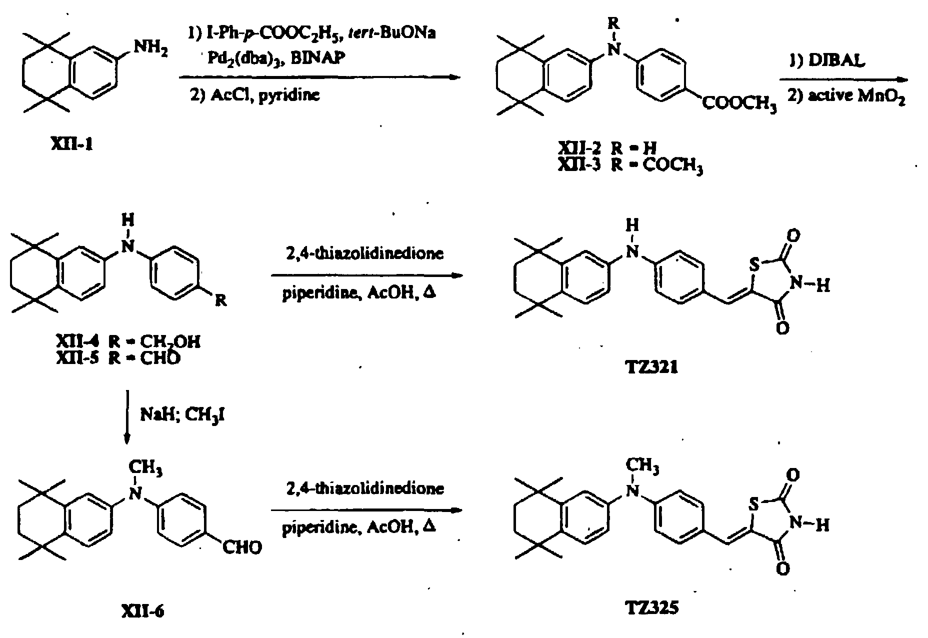

2-Amino-5,6,7,8-tetrahydro-5,5,8,8-tetramethylnaphthalene (XII-1) (1.50 g, 7.39 mmol), ethyl 4-iodobenzoate (1.70 g, 6.16 mmol) and tert-BuONa (0.83 g, 8.62 mmol) were dissolved in anhydrous toluene (30 ml). The mixture was added with tris(dibenzylideneacetone)dipalladium(0)(138 mg, 0.15mmol) and (R)-BINAP (210 mg, 0.33 mmol) under argon atmosphere and then stirred at 80°C. After one hour, the reaction mixture was cooled to room temperature and extracted with ether. The organic layer was washed with brine, dehydrated over MgSO₄, and concentrated. The residue was then purified by silica gel column chromatography (ethyl acetate : n-hexane = 1:8) to obtain compound XII-2 (1.38 g, 64%).
¹H-NMR (400 MHz, CDCl₃): 7.90 (d, 2H, J=8.8Hz), 7.26 (d, 2H, J=8.4Hz), 7.10 (d, 1H, J=2.5Hz), 6.96 (dd, 1H, J=8.4, 2.6Hz), 6.93 (d, 2H, J=8.8Hz), 4.33 (q, 2H, J=7.0Hz), 1.69 (s, 4H), 1.37 (t, 3H, J=7.0Hz), 1.28 (s, 6H), 1.27 (s, 6H).

Compound XII-2 (1.95 g, 5.56 mmol) was dissolved in anhydrous pyridine (10 ml), and the solution was added with acetyl chloride (523 mg, 6.67 mmol) and stirred at room temperature for 3 hours. The reaction mixture was added with ice water and extracted with ethyl acetate. The organic layer was washed with diluted hydrochloric acid and brine, dehydrated over MgSO₄, and concentrated. The residue was then purified by silica gel column chromatography (ethyl acetate : n-hexane = 1:3) to obtain Compound XII-3 (1.34 g, 61.5%).
¹H-NMR (400 MHz, CDCl₃), 8.00 (d, 2H, J=8.4Hz), 7.32 (d, 2H, J=8.8Hz), 7.31 (d, 1H, J=8.8Hz), 7.14 (d, 1H, J=2.2Hz), 6.95 (dd, 1H, J=8.4, 2.2Hz), 4.35 (q, 2H, J=6.9Hz), 2.05 (s, 3H), 1.69 (s, 4H), 1.37 (t, 3H, J=6.9Hz), 1.28 (s, 6H), 1.24 (s, 6H).

Compound XII-3 (1.34 g, 3.41 mmol) was dissolved in THF (6 ml) and gradually added with DIBAL (10.2 ml, 1.0 M solution in toluene, 10.2 mmol) at -78°C. After one hour, the mixture was poured into 1 N hydrochloric acid and extracted with ethyl acetate. After the organic layer was dehydrated over MgSO₄ and concentrated, the residue was purified by silica gel column chromatography (ethyl acetate : n-hexane = 1:2) to obtain Compound XII-4 (621 mg, 59%).
¹H-NMR (400 MHz, CDCl₃): 7.24 (d, 2H, J=8.4Hz), 7.03 (d, 1H, J=2.2Hz), 7.00 (d, 2H, J=8.4Hz), 6.89 (dd, 1H, J=8.4, 2.2Hz), 4.60 (s, 2H), 1.68 (s, 4H), 1.27 (s, 6H), 1.26 (s, 6H).

Compound XII-4 (615 mg, 2.0 mmol) was dissolved in methanol-free methylene chloride (8 ml), and the solution was added with active MnO₂ (2.05 g, 85%, 20.0 mmol) and stirred at room temperature for 16 hours. After the reaction mixture was filtered, the filtrate was concentrated and purified by silica gel column chromatography (ethyl acetate : n-hexane = 1:4) to obtain Compound XII-5 (271 mg, 44%).
¹H-NMR (400 MHz, CDCl₃), 9.78 (s, 1H), 7.73 (d, 2H, J=8.8Hz), 7.29 (d, 1H, J=8.4Hz), 7.11 (d, 1H, J=2.2Hz), 6.99 (m, 3H), 1.70 (s, 4H), 1.29 (s, 6H), 1.28 (s, 6H).

Compound XII-5 (150 mg, 0.49 mmol) and 2,4-thiazolidinedione (63 mg, 0.54 mmol) were suspended in anhydrous toluene (6 ml), and the suspension was added with a solution of piperidine (12.7 mg, 0.15 mmol) and acetic acid (8.9 mg, 0.15 mmol) dissolved in anhydrous toluene (1.5 ml), and then refluxed at 120°C for 30 minutes. The reaction mixture was poured into ice water and extracted with ethyl acetate. The organic layer was washed with brine, dehydrated over MgSO₄, and concentrated to obtain TZ321 (178 mg, 90%).
TZ321: Orange needles (ethyl acetate/n-hexane); mp 297°C; ¹H-NMR (400 MHz, DMSO-d₆, 30°C): 8.69 (s, 1H), 7.65 (s, 1H), 7.42 (d, 2H, J=8.8Hz), 7.26 (d, 1H, J=8.8Hz), 7.07 (d, 1H, J=2.6Hz), 7.06 (d, 2H, J=8.4Hz), 6.98 (dd, 1H, J=8.4, 2.6Hz), 1.64 (s, 4H), 1.24 (s, 6H), 1.24 (s, 6H), Anal. Calcd. for C₂₄H₂₆N₂O₂S, C: 70.91%, H: 6.45%, N:6.89%; Found, C: 71.06%, H: 6.42%, N: 6.88%.

### Example 32: Synthesis of TZ325

NaH (20 mg, 60%, 0.49 mmol) was washed with a small amount of n-hexane, and suspended in DMF (1 ml). The suspension was added with Compound XII-5 (100 mg, 0.33 mmol) dissolved in DMF (4 ml), and stirred at room temperature for 20 minutes. The mixture was added with CH₃I (0.08 ml, 1.28 mmol) and stirred for 30 minutes. After the DMF was evaporated under reduced pressure, the residue was added with water and extracted with methylene chloride. The organic layer was washed with brine, dehydrated over MgSO₄, and concentrated. The residue was purified by silica gel column chromatography (ethyl acetate : n-hexane = 1:5) to obtain Compound XII-6 (80 mg, 76.5%).
¹H-NMR (400 MHz, CDCl₃): 9.75 (s, 1H), 7.68 (d, 2H, J=9.2Hz), 7.34 (d, 1H, J=8.4Hz), 7.14 (d, 1H, J=2.2Hz), 6.96 (dd, 1H, J=8.4, 2.2Hz), 6.76 (d, 2H, J=9.2Hz), 3.37 (s, 3H), 1.71 (s, 4H), 1.31 (s, 6H), 1.26 (s, 6H).

Compound XII-6 (75 mg, 0.23 mmol) and 2,4-thiazolidinedione (30 mg, 0.26 mmol) were suspended in anhydrous toluene (4 ml). The suspension was added with a solution of piperidine (6.0 mg, 0.07 mmol) and acetic acid (12 mg, 0.07mmol) dissolved in anhydrous toluene (0.75 ml), and then refluxed at 120°C. After 30 minutes, the reaction mixture was poured into ice water and extracted with ethyl acetate. The organic layer was washed with brine, dehydrated over MgSO₄, and concentrated. The residue was purified by silica gel column chromatography (ethyl acetate : n-hexane = 1:2) to obtain TZ325 (105 mg, quantitative).
TZ325: Yellow powder (ethyl acetate/n-hexane); mp 238°C; ¹H-NMR (400 MHz, CDCl₃): 8.29 (s, 1H), 7.77 (s, 1H), 7.33, (d, 2H, J=8.2Hz), 7.33 (d, 1H, J=8.4Hz), 7.13 (d, 1H, J=2.6Hz), 6.95 (dd, 1H, J=8.4, 2.6Hz), 6.79 (d, 2H, J=8.8Hz), 3.36 (s, 3H), 1.71 (s, 4H), 1.31 (s, 6H), 1.26 (s, 6H), Anal. Calcd. for C₂₅H₂₈N₂O₂S, C: 71.40%, H:6.71%, N: 6.66%; Found, C: 71.51%, H:6.70%, N: 6.60%.

### Example 33: Synthesis of TZ327

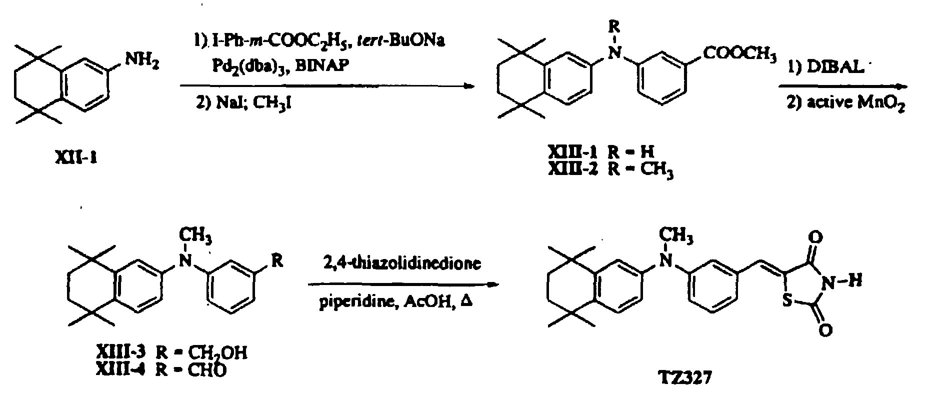

Methyl 3-iodobenzoate (1.24 g, 4.73 mmol), 5,6,7,8-tetrahydro-5,5,8,8-tetramethyl-2-naphthylamine (1.03 g, 5.07 mmol) and tert-BuONa (571 mg, 5.92 mmol) were dissolved in anhydrous toluene (30 ml), and the solution was added with tris(dibenzylideneacetone)dipalladium(0) (117 mg, 0.13 mmol) and (R)-BINAP (177 mg, 0.28 mmol) under argon atmosphere, and stirred at 80°C for one hour. The reaction mixture was cooled to room temperature and extracted with ether. The organic layer was washed with brine, dehydrated over MgSO₄, and then concentrated. The residue was purified by silica gel column chromatography (ethyl acetate : n-hexane = 1:8) to obtain Compound XIII-1 (877 mg, 55%).
¹H-NMR (400 MHz, CDCl₃): 7.70 (t, 1H, 2.0Hz), 7.50 (d, 1H, J=7.7Hz), 7.28 (t, 1H, J=7.9Hz), 7.23 (d, 1H, J=8.4Hz), 7.17 (dd, 1H, J= 8.1, 1.5Hz), 7.06 (d, 1H, J=2.2Hz), 6.90 (dd, 1H, J=8.4, 2.2Hz), 3.89 (s, 3H), 1.69 (s, 4H), 1.28 (s, 6H), 1.27 (s, 6H).

NaH (72 mg, 60%, 1.78 mmol) was washed with n-hexane, dried and suspended in DMF (1 ml). The suspension was added with Compound XIII-1 (400 mg, 1.19 mmol) dissolved in DMF (10 ml), and then stirred at room temperature. After 20 minutes, the mixture was added with methyl iodide (0.28 ml, 4.50 mmol) and stirred for 40 minutes. After DMF was evaporated, the residue was added with water and extracted with methylene chloride. The organic layer was washed with brine. After the solvent was evaporated, the residue was purified by silica gel column chromatography (ethyl acetate : n-hexane = 1:8) to obtain Compound XIII-2 (371.5 mg, 94.5%).
¹H-NMR (400 MHz, CDCl₃): 7.60 (t, 1H, 2.0Hz), 7.47 (d, 1H, 7.7Hz), 7.25 (d, 1H, 8.4Hz), 7.22 (d, 1H, 7.7Hz), 7.08 (d, 1H, 2.6Hz), 7.05 (dd, 1H, 8.4, 2.7Hz), 6.88 (dd, 1H, 8.4Hz, 2.6Hz), 3.88 (s, 3H), 3.33 (s, 3H), 1.68 (s, 4H), 1.29 (s, 6H), 1.24 (s, 6H).

Compound XIII-2 (570 mg, 1.62 mmol) was dissolved in THF (7 ml) under argon atmosphere and the solution was gradually added dropwise with DIBAL (4.87 ml, 1M solution in toluene, 4.87 mmol) at -78°C with stirring. After 30 minutes, the reaction mixture was poured into 2 N hydrochloric acid and extracted with ethyl acetate. The organic layer was washed with 2 N hydrochloric acid, saturated aqueous sodium hydrogencarbonate and brine, dehydrated over MgSO₄, and then concentrated, and the residue was purified by silica gel column chromatography (ethyl acetate : n-hexane = 1:4, then 1:3) to obtain Compound XIII-3 (500 mg, 91%).
¹H-NMR (400 MHz, CDCl₃): 7.23 (d, 1H, 8.3Hz), 7.19 (d, 1H, 8.1Hz), 7.06 (d, 1H, 2.6Hz), 6.94 (br, 1H), 6.88 (dd, 1H, 8.4, 2.2Hz), 6.84 (m, 2H), 4.62 (s, 2H), 3.31 (s, 3H), 1.68 (s, 4H), 1.29 (s, 6H), 1.24 (s, 6H).

Compound XIII-3 (100 mg, 0.30 mmol) was dissolved in methanol-free methylene chloride (4 ml), and the solution was added with active MnO₂ (303 mg, 85%, 2.97 mmol), and stirred at room temperature for 24 hours. The reaction mixture was filtered, and the filtrate was concentrated. Then, the residue was purified by silica gel column chromatography (ethyl acetate : n-hexane = 1:9) to obtain Compound XIII-4 (71.6 mg, 72%).
¹H-NMR (400 MHz, CDCl₃): 9.92 (s, 1H), 7.27-7.38 (m, 4H), 7.10 (d, 1H, 2.6Hz), 7.06-7.09 (m, 1H), 6.92 (dd, 1H, 8.4, 2.2Hz), 3.34 (s, 3H), 1.69 (s, 4H), 1.30 (s, 6H), 1.24 (s, 6H).

Compound XIII-4 (220 mg, 0.66 mmol) and 2,4-thiazolidinedione (84 mg, 0.72 mmol) were suspended in anhydrous toluene (6 ml), and the suspension was added with a solution of piperidine (17 mg, 0.20 mmol) and acetic acid (12 mg, 0.20 mmol) dissolved in anhydrous toluene (2 ml), and refluxed at 120°C for one hour. The reaction mixture was poured into ice water and extracted with ethyl acetate. The organic layer was washed with brine, dehydrated over MgSO₄, and concentrated. The residue was purified by silica gel column chromatography (ethyl acetate : n-hexane = 1:3) to obtain TZ327 (312 mg, quantitative).
TZ327: Orange prisms (ethyl acetate/n-hexane); mp 196°C; ¹H-NMR (400 MHz, CDCl₃): 8.39 (s, 3H), 7.76 (s, 3H), 7.31 (d, 1H, 8.4Hz), 7.27 (d, 1H, 8.4Hz), 7.10 (d, 1H, 2.2Hz), 6.92 (dd, 1H, 8.4Hz, 2.2Hz), 6.89 (d, 2H, 7.0Hz), 6.83 (t, 1H, 2.0Hz), 3.32 (S, 3H), 1.71 (s, 4H), 1.31 (s, 6H), 1.26 (S, 6H); Anal. Calcd. for C₂₅H₂₈N₂O₂S, C: 71.40%, H: 6.71%, N: 6.66%; Found, C: 71.15%, H: 6.61%, N:6.44%.

### Example 34: Synthesis of TZ331

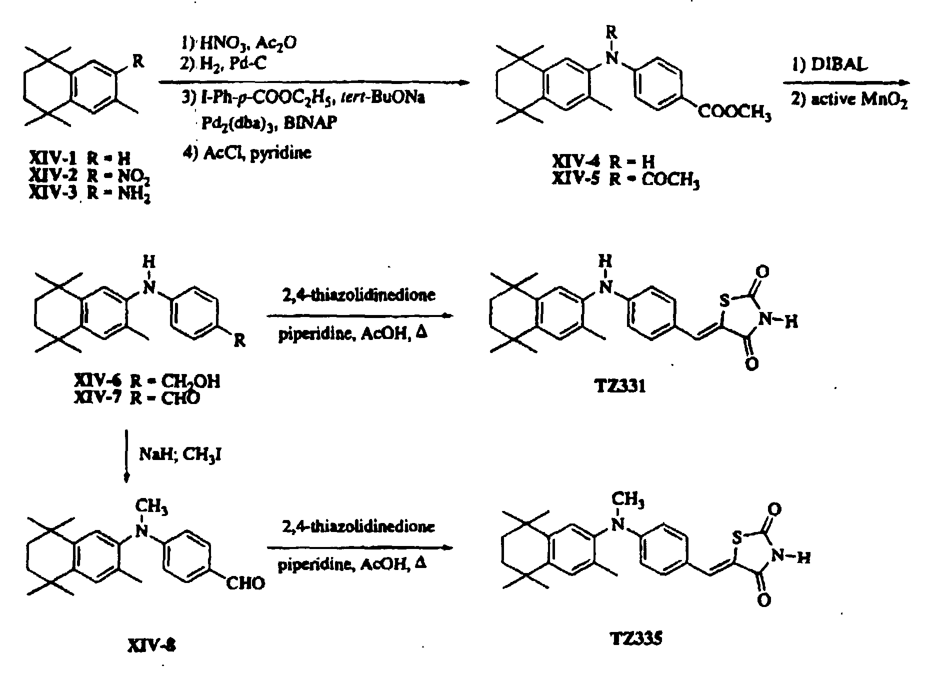

1,2,3,4-Tetrahydro-1,1,4,4,6-pentamethylnaphthalene (2.69 g, 13.3mmol) was dissolved in acetic anhydride (20 ml) and cooled to 0°C. The solution was gradually added with 61% nitric acid (0.74 ml, 16.0 mmol). After 2 hours, the reaction mixture was poured into ice water, neutralized with sodium hydroxide and extracted with ether. The organic layer was washed with brine, dehydrated over MgSO₄, and concentrated to obtain Compound XIV-2 (3.03 g, 92%).
¹H-NMR (400 MHz, CDCl₃): 7.96 (s, 1H), 7.21 (s, 1H), 2.56 (s, 3H), 1.70 (s, 4H), 1.30 (s, 6H), 1.29 (s, 6H).

Compound XIV-2 (3.02 g, 12.2 mmol) was dissolved in ethyl acetate (20 ml) and ethanol (30 ml), and the solution was added with Pd/C (400 mg) and catalytic hydrogen reduction was performed at room temperature. After 6.5 hours, the catalyst was removed by filtration, and the filtrate was concentrated. The residue was purified by silica gel column chromatography (ethyl acetate : n-hexane = 1:4) to obtain Compound XIV-3 (1.48 g, 56%).
¹H-NMR (400 MHz, CDCl₃): 6.97 (s, 1H), 6.61 (s, 1H), 3.45 (br s, 2H), 2.14 (s, 3H), 1.64 (s, 4H), 1.24 (s, 6H), 1.24 (s, 6H).

Methyl 4-iodobenzoate (3.82 g, 13.8 mmol), Compound XIV-3 (3.00 g, 13.8 mmol) and tert-BuONa (1.55 g, 16.1 mmol) were dissolved in anhydrous toluene (30 ml), and the solution was added with tris(dibenzylideneacetone)dipalladium(0) (320 mg, 0.35 mmol) and (R)-BINAP (480 mg, 0.77 mmol) under argon substitution, and stirred at 100°C for 3 hours. The reaction mixture was cooled to room temperature and extracted with ether. The organic layer was washed with brine, dehydrated over MgSO₄, and then concentrated. The residue was purified by silica gel column chromatography (ethyl acetate : n-hexane = 1:10) to obtain Compound XIV-4 (2.04 g, 40%).
¹H-NMR (400 MHz, CDCl₃): 7.89 (d, J=8.8Hz, 2H), 7.21 (s, 1H), 7.18 (s, 1H), 6.76 (d, J=8.8Hz, 2H), 4.32 (q, J=7.0Hz, 2H), 2.19 (s, 3H), 1.68 (s, 4H), 1.37 (t, J=7.0Hz, 3H), 1.29 (s, 6H), 1.24 (s, 6H).

Compound XIV-4 (2.03 g, 5.56 mmol) was dissolved in anhydrous benzene (30 ml), and the solution was added with acetyl chloride (524 mg, 6.67 mmol) and anhydrous pyridine (1 ml), and stirred at room temperature for 2 hours. The reaction mixture was further added with acetyl chloride (0.20 ml) and stirred at 50°C for 4 hours, and then at 60°C for further 23 hours. The reaction mixture was added with ice water and extracted with ethyl acetate. The organic layer was washed with 2 N hydrochloric acid and brine, dehydrated over MgSO₄, and concentrated. The residue was purified by silica gel column chromatography (ethyl acetate : n-hexane = 1:4) to obtain Compound XIV-5 (1.66 g, 62%).
¹H-NMR (400 MHz, CDCl₃): 7.97 (d, J=8.8Hz, 2H), 7.33 (d, J=8.8Hz, 2H), 7.17 (s, 1H), 7.13 (s, 1H), 4.34 (q, J=7.0Hz, 2H), 2.06 (s, 3H), 1.97 (s, 3H), 1.69 (s, 4H), 1.36 (t, J=7.0Hz, 3H), 1.29 (s, 6H), 1.26 (s, 6H).

Compound XIV-5 (1.62 g, 3.98 mmol) was dissolved in THF (10 ml) under argon substitution. This solution was slowly added dropwise with DIBAL (11.9 ml, 1 M solution in toluene, 11.9 mmol) at -78°C with stirring. After 30 minutes, the reaction mixture was poured into 2 N hydrochloric acid and extracted with ethyl acetate. The organic layer was washed with brine, dehydrated over MgSO₄, and concentrated. The residue was purified by silica gel column chromatography (ethyl acetate : n-hexane = 1:2) to obtain Compound XIV-6 (0.99 g, 77%).
¹H-NMR (400 MHz, CDCl₃): 7.23 (d, J=8.4Hz, 2H), 7.19 (s, 1H), 7.12 (s, 1H), 6.87 (d, J=8.4Hz, 2H), 5.33 (s, 1H), 4.60 (d, J=5.5Hz, 2H), 2.20 (s, 3H), 1.67 (s, 4H), 1.51 (t, J=5.6Hz, 1H), 1.28 (s, 6H), 1.22 (s, 6H).

Compound XIV-6 (985 mg, 3.05 mmol) was dissolved in methanol-free methylene chloride (14 ml), and the solution was added with active MnO₂ (3.11 g, 85%, 30.5 mmol), and stirred at room temperature for 22 hours. The reaction mixture was filtered and the filtrate was concentrated. Then, the residue was purified by silica gel column chromatography (ethyl acetate : n-hexane = 1:4) to obtain compound XIV-6 (297 mg, 30%, recovered raw material; 282 mg).
¹H-NMR (400 MHz, CDCl₃): 9.76 (s, 1H), 7.71 (d, J=8.8Hz, 2H), 7.20 (s, 1H), 7.18 (s, 1H), 6.78 (d, J=8.4Hz, 2H), 5.80 (s, 1H), 2.05 (s, 3H), 1.69 (s, 4H), 1.30 (s, 6H), 1.25 (s, 6H).

Compound XIV-6 (70 mg, 0.22 mmol) and 2,4-thiazolidinedione (25.5 mg, 0.22 mmol) were suspended in anhydrous toluene (4 ml), and the suspension was added with a solution of piperidine (5.6 mg, 0.065 mmol) and acetic acid (3.9 mg, 0.065 mmol) dissolved in anhydrous toluene (0.67 ml), and refluxed at 120°C for 7 hours. The reaction mixture was poured into ice water and extracted with ethyl acetate. The organic layer was washed with brine, dehydrated over MgSO₄, and concentrated. The residue was purified by silica gel column chromatography (ethyl acetate : n-hexane = 1:2) to obtain TZ331 (72.5 mg, 79%).
TZ331: Yellow needles (methylene chloride/n-hexane); mp 284°C; ¹H-NMR (400 MHz, CDCl₃): 8.31 (br s, 1H), 7.77 (s, 1H), 7.36 (d, J=8.8Hz, 2H), 7.19 (s, 1H), 7.17 (s, 1H), 6.81 (d, J=8.8Hz, 2H), 5.74 (s, 1H), 2.19 (s, 3H), 1.69 (s, 4H), 1.29 (s, 6H), 1.25 (s, 6H); Anal. Calcd. for C₂₅H₂₈N₂O₂S, C: 71.40%,H: 6.71%, N: 6.66%.

### Example 35: Synthesis of TZ333

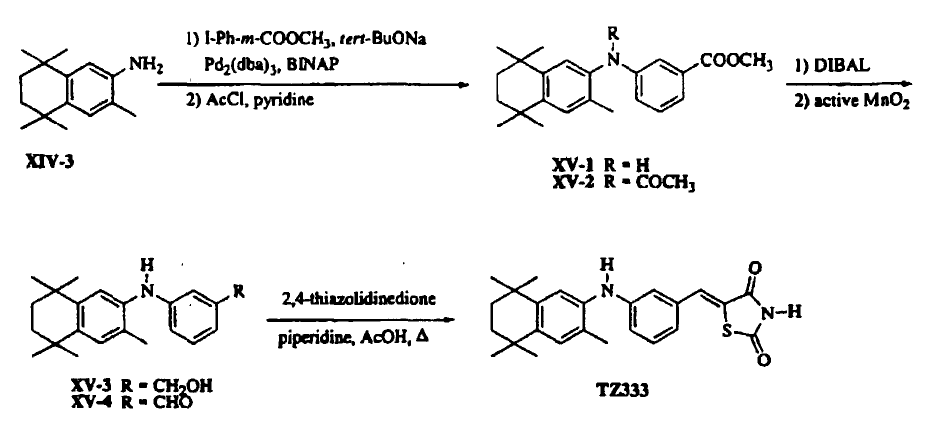

Methyl 3-iodobenzoate (1.77 g, 6.77 mmol), Compound XIV-3 (1.47 g, 6.77 mmol) and tert-BuONa (763 mg, 7.91 mmol) were dissolved in anhydrous toluene (15 ml). The solution was added with tris(dibenzylideneacetone)dipalladium(0) (122 mg, 0.14 mmol) and (R)-BINAP (187 mg, 0.30 mmol) under argon atmosphere, and stirred at 100°C for 2.5 hours. The reaction mixture was cooled to room temperature and extracted with ether. The organic layer was washed with brine, dehydrated over MgSO₄, and concentrated. The residue was purified by silica gel column chromatography (ethyl acetate : n-hexane = 1:8) to obtain Compound XV-1 (1.45 g, 61%).
¹H-NMR (400 MHz, CDCl₃): 7.59 (t, J=2.0Hz, 1H), 7.48 (td, J=7.7, 1.2Hz, 1H), 7.27 (t, J=7.8Hz, 1H), 7.20 (s, 1H), 7.14 (s, 1H), 7.04 (m, 1H), 5.42 (br s, 1H), 3.88 (s, 3H), 2.19 (s, 3H), 1.68 (s, 4H), 1.29 (s, 6H), 1.24 (s, 6H).

Compound XV-1 (1.44 g, 4.10 mmol) was dissolved in anhydrous benzene (16 ml), added with acetyl chloride (386 mg, 4.92 mmol) and anhydrous pyridine (1 ml), and stirred at room temperature for 2 hours. The reaction mixture was added with acetyl chloride (0.20 ml), and stirred at 50°C for 4 hours and then 70°C for further 6 hours. The reaction mixture was added with ice water and extracted with ethyl acetate. The organic layer was washed with 2 N hydrochloric acid and brine, dehydrated over MgSO₄, and concentrated. The residue was purified by silica gel column chromatography (ethyl acetate : n-hexane =1:2) to obtain Compound XV-2 (1.37 g, 85%).
¹H-NMR (400 MHz, CDCl₃): 8.00 (s, 1H), 7.82 (br d, 1H), 7.45 (td, J=8.0, 2.2Hz, 1H), 7.37 (bt, J=8.3Hz, 1H), 7.19 (br s, 1H), 7.15 (s, 1H), 3.88 (s, 3H), 2.10 (s, 3H), 1.96 (s, 3H), 1.69 (s, 4H), 1.27 (s, 12H).

Compound XV-2 (1.37 g, 3.49 mmol) was dissolved in THF (8 ml) under argon substitution and slowly added dropwise with DIBAL (10.5 ml, 1 M solution in toluene, 10.5 mmol) at -78°C with stirring. After 30 minutes, the reaction mixture was poured into 2 N hydrochloric acid and extracted with ethyl acetate. The organic layer was washed with 2 N hydrochloric acid and brine, dehydrated over MgSO₄, and concentrated. The residue was purified by silica gel column chromatography (ethyl acetate : n-hexane = 1:3) to obtain Compound XV-3 (0.91 g, 81%).
¹H-NMR (400 MHz, CDCl₃): 7.21 (t, J=7.7Hz, 1H), 7.20 (s, 1H), 7.12 (s, 1H), 6.92 (s, 1H), 6.82 (m, 2H), 5.35 (br s, 1H), 4.62 (d, J=5.8Hz, 2H), 2.19 (s, 3H), 1.68 (s, 4H), 1.59 (t, J=5.8Hz, 1H), 1.28 (s, 6H), 1.23 (s, 6H).

Compound XV-3 (900 mg, 2.79 mmol) was dissolved in methanol-free methylene chloride (12 ml), and the solution was added with active MnO₂ (2.86 g, 85%, 27.9 mmol) and stirred at room temperature for 15 hours. The reaction mixture was filtered and the filtrate was concentrated. The residue was then purified by silica gel column chromatography (ethyl acetate : n-hexane = 1:8) to obtain Compound XV-4 (119 mg, 13%).
¹H-NMR (400 MHz, CDCl₃): 9.92 (s, 1H), 7.37 (t, J=7.7Hz, 1H), 7.31 (m, 2H), 7.18 (s, 1H), 7.15 (s, 1H), 7.09 (m, 1H), 5.48 (br s, 1H), 2.19 (s, 3H), 1.68 (s, 4H), 1.29 (s, 6H), 1.24 (s, 6H).

Compound XV-4 (115 mg, 0.36 mmol) and 2,4-thiazolidinedione (84 mg, 0.72 mmol) were suspended in anhydrous toluene (8 ml), and the suspension was added with a solution of piperidine (9.2 mg, 0.11 mmol) and acetic acid (6.4 mg, 0.11 mmol) dissolved in anhydrous toluene (1.1 ml), and refluxed at 120°C for 7 hours. The reaction mixture was poured into ice water and extracted with ethyl acetate. The organic layer was washed with brine, dehydrated over MgSO₄, and concentrated. The residue was purified by silica gel column chromatography (ethyl acetate : n-hexane = 1:2) to obtain TZ333 (138 mg, 92%).
TZ333: Yellow needles (ethyl acetate/n-hexane); mp 223°C; ¹H-NMR (400 MHz, CDCl₃), 8.29 (br s, 1H), 7.75 (s, 1H), 7.30 (t, J=8.1Hz, 1H), 7.17 (s, 1H), 7.15 (s, 1H), 6.93 (m, 2H), 6.81 (m, 1H), 5.43 (s, 1H), 2.19 (s, 3H), 1.69 (s, 4H), 1.30 (s, 6H), 1.24 (s, 6H); Anal. Calcd. for C₂₅H₂₈N₂O₂S, C: 71.40%, H: 6.71%, N: 6.66%; Found, C: 71.20%, H: 6.76%, N: 6.65%.

### Example 36: Synthesis of TZ335

NaH (40 mg, 60%, 1.01 mmol) was washed with a small amount of n-hexane and suspended in DMF (1 ml). The suspension was added with XIV-7 (216 mg, 0.67 mmol) dissolved in DMF (6 ml), and stirred at room temperature for 20 minutes. The reaction mixture was added with CH₃I (0.08 ml, 1.35 mmol), and stirred for 30 minutes. After DMF was evaporated under reduced pressure, the residue was added with water and extracted with methylene chloride. The organic layer was washed with brine, dehydrated over MgSO₄, and concentrated. The residue was purified by silica gel column chromatography (ethyl acetate : n-hexane = 1:4) to obtain XIV-8 (140 mg, 62%).
¹H-NMR (400 MHz, CDCl₃): 9.73 (s, 1H), 7.67 (D, J=8.1Hz, 2H), 7.20 (S, 1H), 7.03 (s, 1H), 6.54 (br s, 2H), 3.30 (S, 3H), 2.04 (S, 3H), 1.69 (S, 4H), 1.31 (s, 6H), 1.23 (s, 6H).

XIV-8 (130 mg, 0.39 mmol) and 2,4-thiazolidinedione (45 mg, 0.39 mmol) were suspended in anhydrous toluene (6 ml), and the suspension was added with a solution of piperidine (9.9 mg, 0.12 mmol) and acetic acid (7 mg, 0.12 mmol) dissolved in anhydrous toluene (1.2 ml), and then refluxed at 120°C. After 6 hours, the reaction mixture was poured into ice water and extracted with methylene chloride. The organic layer was washed with brine, dehydrated over MgSO₄, and concentrated. The residue was purified by silica gel column chromatography (ethyl acetate : n-hexane = 1:3) to obtain TZ335 (145 mg, 86%).
TZ335: Yellow powder (methylene chloride/methanol); mp >300°C; ¹H-NMR (400 MHz, DMSO-d₆, 30°C): 12.30 (br s, 1H), 7.63 (S, 1H), 7.39 (d, J=8.4Hz, 2H), 7.29 (s, 1H), 7.09 (s, 1H), 6.53 (d, J=8.3Hz, 2H), 3.29 (s, 3H), 1.99 (s, 3H), 1.65 (s, 4H), 1,27 (s, 6H), 1.21 (s, 6H), Anal. Calcd. for C₂₆H₃₀N₂O₂S, C: 71.86%, H: 6.96%, N: 6.45%; Found, C: 71.60%, H:6.99%, N: 6.67%.

### Example 37: Synthesis of TZ337

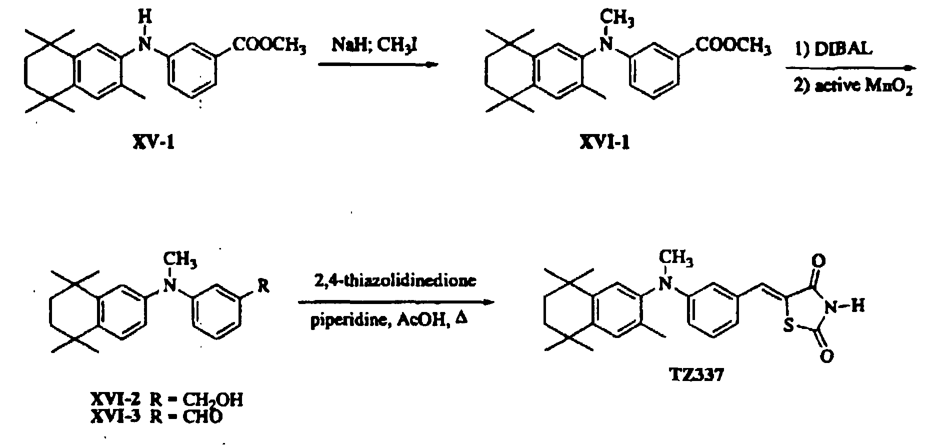

NaH (146 mg, 60%, 3.65 mmol) was washed with a small amount of n-hexane, and suspended in DMF (1 ml). The suspension was added with XV-1 (855 mg, 2.44 mmol) dissolved in DMF (12 ml), and stirred at room temperature for 20 minutes. The reaction mixture was added with CH₃I (0.30 ml, 4.87 mmol) and stirred for one hour. After DMF was evaporated under reduced pressure, the residue was added with water and extracted with methylene chloride. The organic layer was washed with brine, dehydrated over MgSO₄, and concentrated. The residue was purified by silica gel column chromatography (ethyl acetate : n-hexane = 1:10) to obtain XVI-1 (788.5 mg, 89%).
¹H-NMR (400 MHz, CDCl₃): 7.34 (d, J=7.7Hz, 1H), 7.30 (m, 1H), 7.17 (s, 1H), 7.16 (t, J=7.7Hz, 1H), 7.04 (s, 1H), 6.59 (dd, J=7.4, 1.8Hz, 1H), 3.88 (s, 3H), 3.25 (s, 3H), 2.04 (s, 3H), 1.68 (s, 4H), 1.30 (s, 3H), 1.22 (s, 3H).

XVI-1 (750 mg, 2.05 mmol) was dissolved in THF (7 ml) under argon atmosphere and gradually added dropwise with DIBAL (6.16 ml, 1 M solution in toluene, 6.16 mmol) at -78°C with stirring. After 30 minutes, the reaction mixture was poured into 2 N hydrochloric acid and extracted with ethyl acetate. The organic layer was washed with brine, dehydrated over MgSO₄, and concentrated. The residue was purified by silica gel column chromatography (ethyl acetate : n-hexane = 1:2) to obtain XVI-2 (616 mg, 89%).
¹H-NMR (400 MHz, CDCl₃): 7.16 (s, 1H), 7.14 (t, J=7.7Hz, 1H), 7.04 (s, 1H), 6.68 (d, J=7.3Hz, 1H), 6.58 (s, 1H), 6.41 (dd, J=8.1, 2.2Hz, 1H), 4.60 (d, J=5.8Hz, 2H), 3.22 (s, 3H), 2.06 (s, 3H), 1.68 (s, 4H), 1.52 (t, J=5.9Hz, 1H), 1.30 (s, 6H), 1.21 (s, 6H).

Compound XVI-2 (610 mg, 1.81 mmol) was dissolved in methanol-free methylene chloride (8 ml), and the solution was added with active MnO₂ (1.85 g, 85%, 18.1 mmol) and stirred at room temperature for 30 hours. After the reaction mixture was filtered, the filtrate was concentrated and the residue was purified by silica gel column chromatography (ethyl acetate : n-hexane = 1:10) to obtain XV-3 (423 mg, 70%).
¹H-NMR (400 MHz, CDCl₃): 9.91 (s, 1H), 7.28 (t, J=7.3Hz, 1H), 7.18 (m, 2H), 7.07 (m, 1H), 7.04 (s, 1H), 6.69 (dd, J=8.4, 2.6Hz, 1H), 3.20 (s, 3H), 2.05 (s, 3H), 1.69 (s, 4H), 1.31 (s, 6H), 1.22 (s, 6H).

XV-3 (415 mg, 1.24 mmol) and 2,4-thiazolidinedione (145 mg, 1.42 mmol) were suspended in anhydrous toluene (10 ml), and the suspension was added with a solution of piperidine (32 mg, 0.37mmol) and acetic acid (22 mg, 0.37 mmol) dissolved in anhydrous toluene (4 ml), and then refluxed at 120°C. After 6 hours, the reaction mixture was poured into ice water and extracted with ethyl acetate. The organic layer was washed with brine, dehydrated over MgSO₄, and concentrated. The residue was purified by silica gel column chromatography (ethyl acetate : n-hexane = 1:3) to obtain TZ337 (504 mg, 94%).
TZ337: Orange crystals (ethyl acetate/n-hexane); mp 219°C; ¹H-NMR (400 MHz, CDCl₃): 8.22 (br s, 1H), 7.74 (s, 1H), 7.27 (t, J=7.7Hz, 1H), 7.04 (s, 1H), 6.80 (d, J=8.4Hz, 1H), 6.64 (dd, J=8.0, 2.2Hz, 1H), 6.48 (s, 1H), 3.26 (S, 3H), 2.05 (S, 3H), 1.70 (s, 4H), 1.32 (s, 6H), 1.24 (s, 6H); Anal. Calcd. for C₂₆H₃₀N₂O₂S, C: 71.86%, H: 6.96%, N: 6.45%; Found, C: 71.65%, H: 7.16%, N:6.75%.

### Example 38: Test Example

Cell differentiation-inducing activity of each compound of the present invention used alone, and effect on cell differentiation-inducing activity of coexisting retinoid were examined. As a comparative or coexisting retinoid, Am80 ([4-[(5,6,7,8-tetrahydro-5,5,8,8-tetramethyl-2-naphthalenyl)carbamoyl]benzoic acid] was used. Promyelocytic leukemia cell strain HL-60 was used, and differentiation into granulocytic cells was determined by observing morphological change and measuring ability to reduce nitroblue tetrazolium (NBT). The ratios of differentiated cells shown in the following tables were calculated from the ability to reduce NBT.
(A) Concentration-dependent differentiation-inducing ability of each compound alone and concentration-dependent effect on the differentiation-inducing ability of 1×10⁻⁹ M Am80 were measured. Each of TZ91 and TZ181, per se, had cell differentiation ability, and enhanced the activity of the coexisting Am80 at a concentration which did not give the cell differentiation-inducing ability. TZ201 itself was found to be inactive, whilst it suppressed the activity of the coexisting Am80.

**Table 1**

| Compound | Ratio of differentiation-induced cells by treatment with each compound alone (%) | | | | Ratio of differentiation-induced cells with coexistence of 1×10⁻⁹ Am80 (%) | | | | |
|---|---|---|---|---|---|---|---|---|---|
| | Concentration | | | | Concentration | | | | |
| | -9 | -8 | -7 | -6 | None | -9 | -8 | -7 | -6 |
| TZ91 | 1.2 | 0.8 | 7 | 87 | 49 | 58 | 62 | 87 | - |
| TZ181 | - | 1 | 7 | 54 | 37 | - | 53 | 58 | 6 |
| TZ201 | - | 0.3 | 0.7 | 0.3 | 48 | - | 64 | 53 | 5 |

(B) Concentration-dependent differentiation-inducing ability of each compound alone and concentration-dependent effect on the differentiation inducing ability of 1×10⁻¹⁰ M Am80 were measured. TZ151, per se, had cell differentiation ability, and enhanced the activity of the coexisting Am80 at a concentration which did not give the cell differentiation inducing ability. TZ161 and TZ191 per se were found to be inactive, they enhanced the activity of the coexisting Am80, and acted to suppress the activity at a high concentration (1×10⁻⁶ M).

**Table 2**

| Compound | Ratio of differentiation-induced cells by treatment with each compound alone(%) | | | Ratio of differentiation-induced cells with coexistence of 1×10⁻¹⁰ Am80 (%) | | | |
|---|---|---|---|---|---|---|---|
| | Concentration | | | Concentration | | | |
| | -8 | -7 | -6 | None | -8 | -7 | -6 |
| TZ151 | 3 | 4.4 | 78 | 4 | 12 | 43 | 83 |
| TZ161 | 3.5 | 1.8 | 3.6 | 4 | 12 | 25 | 3.8 |
| TZ191 | 3.6 | 3.5 | 4.1 | 11 | 63 | 75 | 28 |

(C) Concentration-dependent differentiation-inducing ability of each compound alone and concentration-dependent effect on the differentiation inducing ability of 3×10⁻⁹ M Am80 were measured. Tested compounds except for TZ241 had cell differentiation ability when used alone, and all of the five compounds enhanced the activity of the coexisting Am80 at a concentration which did not give the cell differentiation inducing ability.

**Table 3**

| Compound | Ratio of differentiation-induced cells by treatment with each compound alone(%) | | | Ratio of differentiation-induced cells with coexistence of 3×10⁻⁹ Am80 | | | |
|---|---|---|---|---|---|---|---|
| | Concentration | | | Concentration | | | |
| | -8 | -7 | -6 | None | -8 | -7 | -6 |
| TZ221 | 1.4 | 2 | 51 | 44 | 54 | 67 | 82 |
| TZ241 | 2.8 | 6.4 | 89 | 44 | 76 | 84 | 92 |
| TZ245 | 3.8 | 3 | 11 | 44 | 86 | 89 | 88 |
| TZ321 | 1.2 | 1.1 | 28 | 51 | 55 | 83 | 88 |
| TZ325 | 2.2 | 21 | 87 | 51 | 72 | 83 | 79 |

(D) Effect on concentration-dependent differentiation-inducing ability of the retinoid Am80 was measured with a fixed concentration of each compound at 1×10⁻⁶ M. Each of the following four compounds did not have cell differentiation ability when used alone, and suppressed the activity of the coexisting Am80.

**Table 4**

| | Coexisting retinoid (concentration) | | | |
|---|---|---|---|---|
| Compound | None | Am80 (-9) | Am80 (-9.5) | Am80 (-10) |
| None | 1.5 | 80 | 53 | 8.5 |
| TZ223 | 4.4 | 62 | 22 | 5 |
| TZ227 | 5.3 | 11.7 | 5.5 | 7 |
| TZ243 | 4.2 | 77 | 35 | 5 |
| TZ247 | 7 | 10 | 5.8 | 6.4 |

(E) Japanese Patent Unexamined Publication No. (Hei)9-48771/1997 discloses that N-benzyldioxothiazolidylbenzamide derivatives represented by the following general formula have an action of improving insulin resistance. For comparison, TZ105 as an N-benzyl derivative was synthesized, and examined whether or not the compound has the retinoid activities.

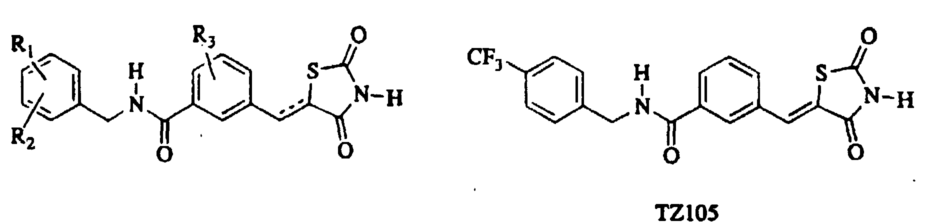

Compound II-2 (see, Example 4, 150 mg, 0.60 mmol) was suspended in anhydrous benzene (12 ml), and the suspension was added with SOCl₂ (358 mg, 3.01 mmol), and refluxed for 14 hours. After SOCl₂ was evaporated, the residue was suspended in anhydrous benzene (10 ml), and the suspension was added with 4-trifluorobenzylamine (106 mg, 0.60 mmol) and anhydrous pyridine (1 ml), and stirred at room temperature for 1 hour. The reaction mixture was added with 2 N hydrochloric acid with floating ice, and extracted with ethyl acetate. The organic layer was washed with brine, dehydrated over MgSO₄, and concentrated. The residue was purified by silica gel column chromatography (ethyl acetate : n-hexane = 3:2) to obtain TZ105 (128 mg, 52 %).
TZ105: Colorless needles (ethyl acetate/n-hexane); mp 204°C; ¹H-NMR (400 MHz; DMSO-d₆, 30°C) 9.23 (t, 1H, J=5.9Hz), 8.10 (s, 1H), 7.97 (d, 1H, J=8.7Hz), 7.83 (s, 1H), 7.76 (d, 1H, J=8.7Hz), 7.70 (d, 2H, J=8.1Hz), 7.65 (t, 1H, J=7.7Hz), 7.55 (d, 2H, J=8.0Hz), 4.59 (d, 2H, J=5.9Hz); Anal. Calcd. for C₁₉H₁₃N₂O₃SF₃, C: 56.16%, H: 3.22%, N: 6.89%, Found, C: 56.36%, H: 3.04%, N: 6.98%

In the aforementioned assay system using HL-60 cells, TZ105 was completely inactive in the differentiation inducing activity, and also failed to effect the activity of the coexisting retinoid Am80. Therefore, it is considered that N-benzyl compounds have no retinoid activity or no activity of controlling retinoids, and the presence of an aromatic ring on the nitrogen atom, such as seen in TZ185 and the like, is essential in the skeleton.

### Industrial Applicability

The compounds of present invention act on the retinoid receptor and have retinoid-like activities, action of controlling retinoids (enhancement or suppression of retinoid activities) and the like. Therefore, they are useful as active ingredients of medicaments for preventive and/or therapeutic treatments of diseases such as cancers, diabetes, arteriosclerosis, bone diseases, rheumatism, immunological diseases and the like.

## Claims

1. A compound represented by the following general formula (I): 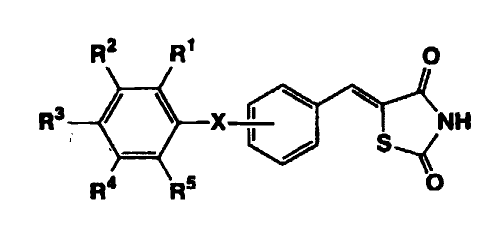 wherein R¹, R², R³, R⁴, and R⁵ independently represent hydrogen atom or a lower alkyl group, or two adjacent groups selected therefrom may combine together with carbon atoms of the phenyl ring to which they bind to form a 5- or 6-membered ring which may optionally be substituted with one or more alkyl groups; and X represents a group represented by -C(R⁶)=CH-, -CH=C(R⁷)-, -N(R⁸)CO-, -CO-N(R⁹)-, -C(=CHR¹⁰), -CO-, or -NR¹¹- wherein R⁶, R⁷, R⁸, R⁹, R¹⁰, and R¹¹ independently represent hydrogen atom or a lower alkyl group, or
a compound represented by the following general formula (II): 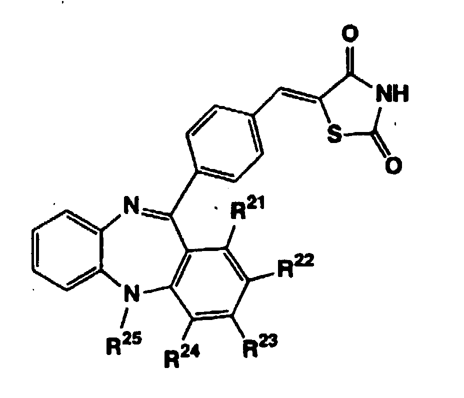 wherein R²¹, R²², R²³, and R²⁴ independently represent hydrogen atom or a lower alkyl group, or two adjacent groups selected therefrom may combine together with carbon atoms of the phenyl ring to which they bind to form a 5- or 6-membered ring which may optionally be substituted with one or more alkyl groups; and R²⁵ represents hydrogen atom or a lower alkyl group, or a salt thereof.

2. A medicament comprising a substance selected from the group consisting of a compound represented by the formula (I) or formula (II) according to claim 1, a physiologically acceptable salt thereof, and a hydrate thereof and solvate thereof.

3. The medicament according to claim 2, which is an agent acting on a retinoid receptor.

4. The medicament according to claim 2 or 3, which has an action of enhancing an activity of a retinoid.

5. The medicament according to claim 2 or 3, which has an action of suppressing an activity of a retinoid.
